(19) Europäisches Patentamt
European Patent Office
Office européen des brevets

(11) EP 4 748 903 A1

(12) EUROPEAN PATENT APPLICATION
published in accordance with Art. 153(4) EPC

(43) Date of publication:
**27.05.2026 Bulletin 2026/22**

(21) Application number: **24845215.3**

(22) Date of filing: **06.06.2024**

(51) International Patent Classification (IPC):
***C09K 3/00*** *(2006.01)* ***A23L 33/10*** *(2016.01)*
***A23L 33/105*** *(2016.01)* ***A61K 8/36*** *(2006.01)*
***A61K 31/202*** *(2006.01)* ***A61Q 19/00*** *(2006.01)*
***C07C 51/47*** *(2006.01)* ***C07C 51/353*** *(2006.01)*
***C07C 59/76*** *(2006.01)* ***C09K 15/06*** *(2006.01)*

(52) Cooperative Patent Classification (CPC):
**A23L 33/10; A23L 33/105; A61K 8/36; A61K 31/202; A61Q 19/00; C07C 51/353; C07C 51/47; C07C 59/76; C09K 3/00; C09K 15/06**

(86) International application number:
**PCT/JP2024/020712**

(87) International publication number:
**WO 2025/022833 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.07.2023 JP 2023118923**

(71) Applicant: **Meijo University**
**Nagoya-shi, Aichi 468-8502 (JP)**

(72) Inventor: **KAGEYAMA Hakuto**
**Nagoya-shi, Aichi 468-8502 (JP)**

(74) Representative: **Müller-Boré & Partner Patentanwälte PartG mbB**
**Friedenheimer Brücke 21**
**80639 München (DE)**

(54) **ULTRAVIOLET ABSORBER, ANTIOXIDANT, ANTI-GLYCATION AGENT, EXTERNAL PREPARATION FOR SKIN, COSMETIC, METHOD FOR PRODUCING COMPOUND, AND COMPOUND**

(57) Provided are a novel ultraviolet absorber, a novel antioxidant, or a novel anti-glycation agent.

An ultraviolet absorber, an antioxidant, or an anti-glycation agent containing, as an active ingredient, at least one compound of a compound represented by the following formula (1) and a compound represented by the following formula (2).

EP 4 748 903 A1

[Chemical Formula 1]

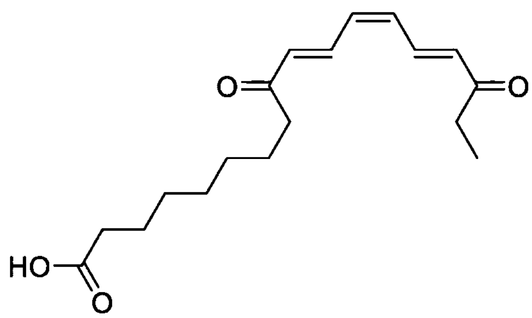

(1)

[Chemical Formula 2]

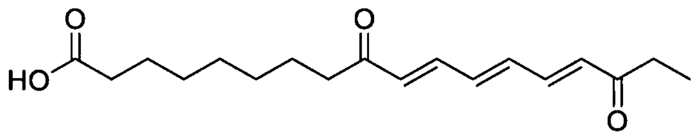

(2)



(Cont. next page)

Fig. 7

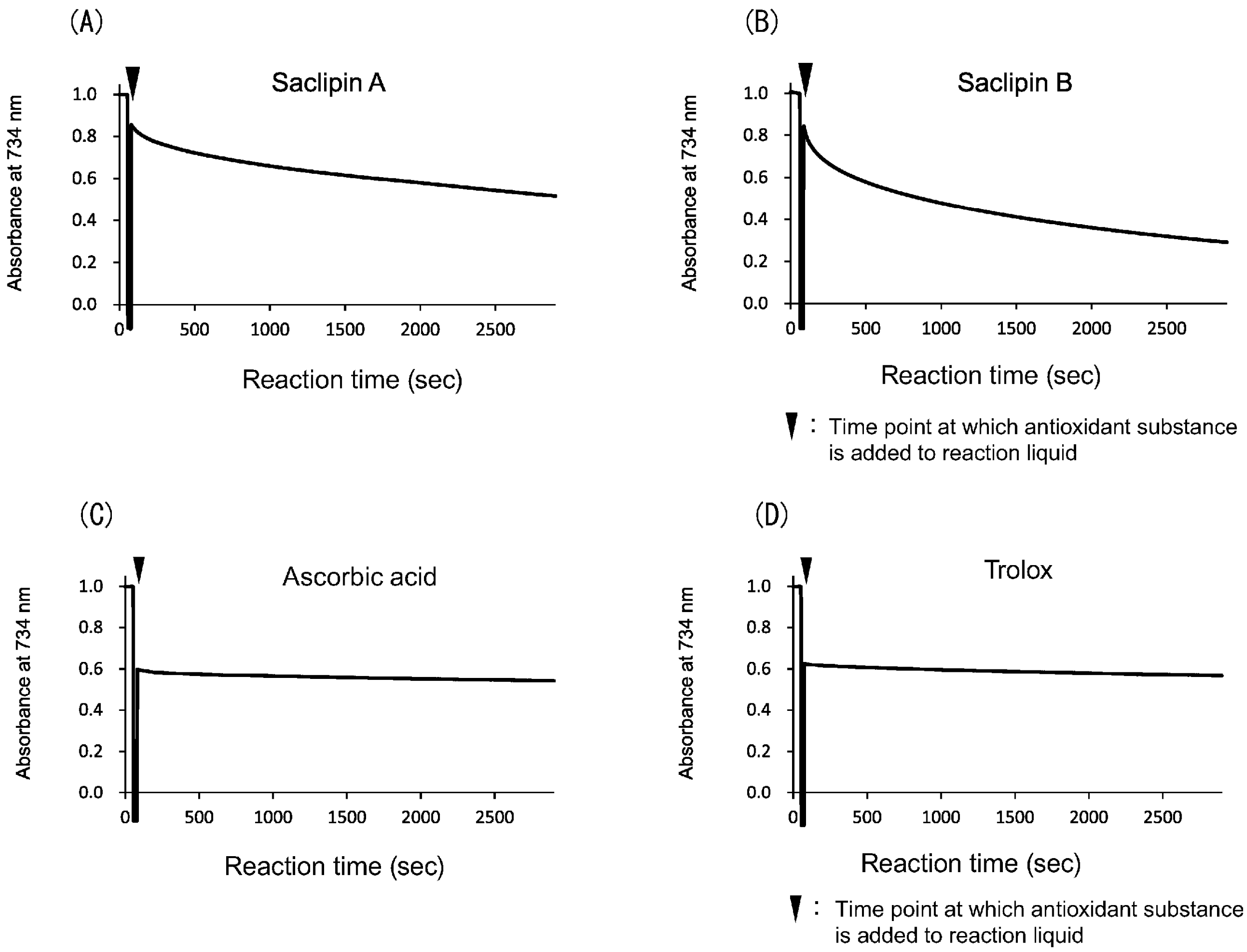
(A)
Saclipin A
Absorbance at 734 nm
1.0
0.8
0.6
0.4
0.2
0.0
0
500
1000
1500
2000
2500
Reaction time (sec)
(B)
Saclipin B
Absorbance at 734 nm
Reaction time (sec)
▼ : Time point at which antioxidant substance is added to reaction liquid
(C)
Ascorbic acid
Absorbance at 734 nm
Reaction time (sec)
(D)
Trolox
Absorbance at 734 nm
Reaction time (sec)
▼ : Time point at which antioxidant substance is added to reaction liquid

## Description

TECHNICAL FIELD

The present disclosure relates to an ultraviolet absorber, an antioxidant, an anti-glycation agent, an external preparation for skin, a cosmetic, a method for producing a compound, and a compound.

BACKGROUND ART

Patent Literature 1 describes a compound referred to as junsainoside A as a useful novel compound contained in junsai.

CITATIONS LIST

PATENT LITERATURE

Patent Literature 1: JP 2014-31361 A

SUMMARY OF INVENTION

TECHNICAL PROBLEMS

The inventor of the present application has previously studied an ultraviolet absorbing substance derived from cyanobacteria. As the ultraviolet absorbing substance biosynthesized by cyanobacteria, mycosporine-like amino acids and scytonemin are well known.

An object of the present disclosure is to solve at least one of providing a novel ultraviolet absorber, a novel antioxidant, a novel anti-glycation agent, a novel external preparation for skin, and a novel cosmetic, providing a novel method for producing a compound, and providing a novel compound.

SOLUTIONS TO PROBLEMS

In searching for a substance different from mycosporine-like amino acids and sitonemin, the inventor of the present application has focused on Suizenji-nori which is edible cyanobacteria unique to Japan. Then, the present inventor has newly found that there is a substance having strong absorption in an ultraviolet region as a result of analyzing an extraction liquid of Suizenji-nori, and has developed the technology of the present disclosure. The present disclosure can be implemented in the following forms.

[1] An ultraviolet absorber, an antioxidant, or an anti-glycation agent containing, as an active ingredient, at least one compound of a compound represented by the following formula (1) and a compound represented by the following formula (2).

[Chemical Formula 1]

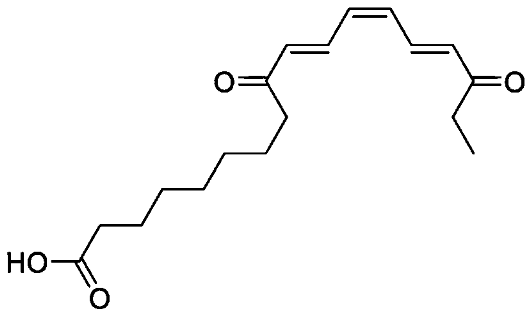

(1)

[Chemical Formula 2]

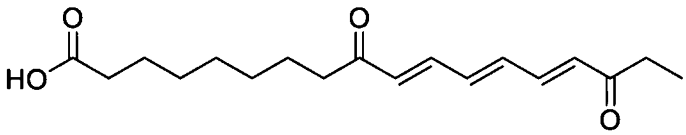

(2)

[2] An external preparation for skin or a cosmetic containing at least one compound of a compound represented by the above formula (1) and a compound represented by the above formula (2).

[3] A method for producing a compound, including extracting at least one compound of a compound represented by the above formula (1) and a compound represented by the above formula (2) from cyanobacteria.

[4] The method for producing a compound according to [3], further including drying the cyanobacteria before the extracting of the compound.

[5] A method for producing a compound, including irradiating a solution of a compound represented by the above formula (2) with light to obtain a compound represented by the above formula (1).

[6] At least one compound selected from the group consisting of a derivative of a compound represented by the above formula (1) and a derivative of a compound represented by the above formula (2).

[7] An ultraviolet absorber, an antioxidant, or an anti-glycation agent containing the compound according to [6] as an active ingredient.

[8] An external preparation for skin or a cosmetic containing the compound according to [6].

BRIEF DESCRIPTION OF DRAWINGS

Fig. 1 is a chromatogram obtained by performing HPLC analysis of an extraction liquid of Suizenji-nori.
Fig. 2 is a diagram for explaining a hypothesis regarding stability of saclipin B.
Fig. 3 shows absorption spectra of saclipin A and saclipin B.
Fig. 4 is a diagram showing main HMBC correlation and NOE correlation of saclipin A.
Fig. 5 is a diagram showing main HMBC correlation and NOE correlation of saclipin B.
Fig. 6 shows chromatograms obtained by HPLC analysis of a saclipin A solution before and after light irradiation.

Fig. 7 shows graphs showing the temporal changes of the antioxidant activity of saclipin A, saclipin B, ascorbic acid, and Trolox.

Fig. 8 is a graph showing an inhibitory effect on the glycation reaction of elastin.

Fig. 9 is a graph showing an inhibitory effect on the glycation reaction of collagen.

Fig. 10 shows chromatograms obtained by HPLC analysis of saclipin A and saclipin B before and after methyl esterification.

Fig. 11 shows absorption spectra of saclipin A and saclipin B before and after methyl esterification.

Fig. 12 is a diagram showing the results of a biocompatibility test on neonatal human dermal fibroblasts.

Fig. 13 is a diagram showing the results of a biocompatibility test on human keratinocytes.

DESCRIPTION OF EMBODIMENT

Hereinafter, a present embodiment will be described in detail. In the present specification, the description using "to" for a numerical range includes the lower limit value and the upper limit value unless otherwise specified. For example, the expression "10 to 20" includes both the lower limit value "10" and the upper limit value "20". That is, "10 to 20" has the same meaning as "10 or more and 20 or less". In the present specification, the upper limit value and the lower limit value of each numerical range can be arbitrarily combined.

1. Compound represented by formula (1) and compound represented by formula (2)

The present disclosure relates to a compound represented by the following formula (1).

[Chemical Formula 3]

Saclipin B

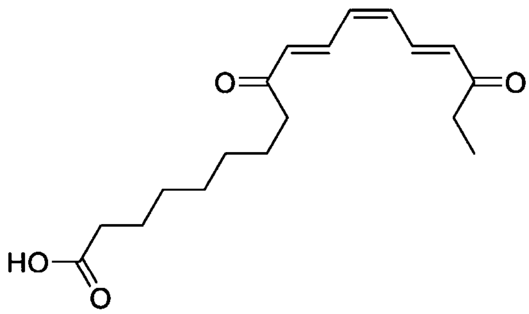

(1)

The compound represented by the formula (1) is (10E,12Z,14E)-9,16-Dioxooctadeca-10,12,14-trienoic acid in IUPAC nomenclature. The molecular weight of the present compound is 306. The inventor of the present application named the present compound as saclipin B.

Suizenji-nori (Aphanothece sacrum) is known as cyanobacteria unique to Japan, and is found to inhabit only in a part of Kyushu. At present, it is grown naturally only in a fish farm using the Kogane River in Asakura City, Fukuoka Prefecture, and is distributed as food.

The inventor of the present application has found that there is a substance having strong absorption in an ultraviolet region as a result of analyzing an extraction liquid of Suizenji-nori. A substance having strong absorption in an ultraviolet region was isolated and purified by preparative HPLC, and the structure of the purified product was analyzed, and as a result, it was found to be a known compound of the following formula (2) and a compound of the above formula (1).

[Chemical Formula 4]

Saclipin A

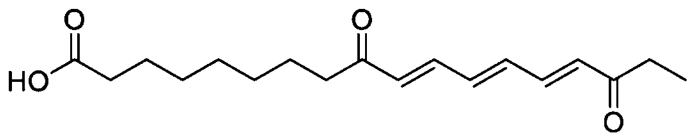

(2)

The compound represented by the formula (2) is (10E,12E,14E)-9,16-Dioxooctadeca-10,12,14-trienoic acid in IUPAC nomenclature. The molecular weight of the present compound is 306. The inventor of the present application named the present compound as saclipin A. Saclipin A and saclipin B are in a geometric isomer (cis-trans isomer) relationship. The compound represented by the formula (2) is a known compound, but it has not been reported that the compound is present in Suizenji-nori or has physiological activity.

Fig. 1 is a chromatogram obtained by performing HPLC analysis of an extraction liquid of Suizenji-nori. The measurement conditions of HPLC analysis are as follows. In Fig. 1, a peak at a retention time of 10 min to 12 min is a peak of saclipin A, and a peak at a retention time of 12 min to 14 min is a peak of saclipin B.

[Measurement conditions]

Detection wavelength: 320 nm
Column: Inerteil ODS-P 3 μm, 4.6×33 + 4.6×150 mm
Eluent: 50% Acetonitrile (isocratic)

Thermodynamically, an organic compound having a carbon-carbon double bond is generally said to be more stable in the trans-form (E-form) than in the cis-form (Z-form). It is surprising that not only saclipin A of the trans-form (E-form) but also saclipin B of the cis-form (Z-form) are present in Suizenji-nori. The reason why saclipin B can stably exist is not clear, but as shown in Fig. 2, in the enol form of saclipin B, there is a possibility that saclipin B is stabilized by the action of a hydrogen bond. Saclipin B is a compound useful for various applications also from the viewpoint of stability.

In Suizenji-nori, the ratio of saclipin A and saclipin B is usually about 75:35 to 90:10. The inventor of the present application has obtained a new finding that an isomerization reaction from saclipin A to saclipin B proceeds by light irradiation. The light irradiation processing will be described later. The ratio of saclipin A and saclipin B can be appropriately designed according to the application and the like. For example, the ratio of saclipin A and saclipin B can be 70:30 to 5:95, and can also be 50:50 to 8:92 or 30:70 to 10:90.

As described above, saclipin B can be obtained by extracting from Suizenji-nori. In addition, saclipin A can also be obtained by extracting from Suizenji-nori. A method for producing saclipin B and saclipin A will be described later. The method for producing saclipin B and saclipin A is not limited thereto. Saclipin B and saclipin A may be produced by synthesizing the compounds on the basis of a known chemical synthesis method, or may be produced by performing a treatment such as a reaction using a substance obtained from a natural product as a raw material.

2. Utilization of compound (part 1)

A first aspect of the present disclosure is an ultraviolet absorber, an antioxidant, or an anti-glycation agent containing, as an active ingredient, at least one compound of the compound represented by the above formula (1) and the compound represented by the above formula (2). Hereinafter, at least one compound of the compound represented by the above formula (1) and the compound represented by the above formula (2) is also referred to as saclipin B and/or saclipin

A. In addition, saclipin B and saclipin A are collectively referred to simply as saclipin without distinction.

Saclipin B has absorption maximum at 319 nm in the ultraviolet wavelength range, and has a molar absorption coefficient at the absorption maximum of 30,555 $M^{-1}cm^{-1}$. Saclipin A has absorption maximum at 315 nm to 316 nm in the ultraviolet wavelength range, and has a molar absorption coefficient at the absorption maximum of 26,454 $M^{-1}cm^{-1}$. Saclipin B and/or saclipin A has a sufficient ultraviolet absorbing action, and is useful as an active ingredient of an ultraviolet absorber. Saclipin B has a molar absorption coefficient at the absorption maximum higher than that of saclipin A. Therefore, the ultraviolet absorber preferably contains at least saclipin B.

The form of the ultraviolet absorber is not particularly limited. The form of the ultraviolet absorber is suitable as an external preparation for skin, a cosmetic, a food and drink, a pharmaceutical, a quasi-pharmaceutical product, and the like. The ultraviolet absorber can effectively exert its action and effect particularly by being applied to the skin surface. Therefore, among the above forms, the form is particularly suitable as an external preparation for skin or a cosmetic.

In addition, saclipin B and/or saclipin A has an antioxidative action, and is also useful as an active ingredient of an antioxidant. Saclipin B has higher radical scavenging activity than saclipin A. Therefore, the antioxidant preferably contains at least saclipin B.

Saclipin B and saclipin A are slow-acting antioxidants capable of maintaining antioxidant activity for a long time as compared with ascorbic acid and Trolox (6-hydroxy-2,5,7,8-tetramethylchroman-2-carboxylic acid). Hereinafter, a specific description will be given. In the measurement of the ABTS radical scavenging activity in Examples described later, when the $IC_{50}$ value after 20 minutes from the time point at which the antioxidant substance was added to the reaction liquid is $\alpha$ and the $IC_{50}$ value after 40 minutes therefrom is $\beta$, the respective $\beta/\alpha$ values of saclipin B, saclipin A, ascorbic acid, and Trolox are as follows. Ascorbic acid and Trolox have a $\beta/\alpha$ value of 0.95 or more, whereas saclipin B and/or saclipin A has a $\beta/\alpha$ value of 0.8 or less. Note that the $\beta/\alpha$ value is usually 1 or less, and a smaller $\beta/\alpha$ value serves as an indicator that the radical scavenging reaction has progressed in 20 minutes to 40 minutes.

$\beta/\alpha$ value of saclipin B = 0.74
$\beta/\alpha$ value of saclipin A = 0.78
$\beta/\alpha$ value of ascorbic acid = 0.95
$\beta/\alpha$ value of Trolox = 1.00

The form of the antioxidant is not particularly limited. The form of the antioxidant is suitable for a food and drink and a pharmaceutical. According to such a form, active oxygen can be scavenged in a living body in which the antioxidant has been orally ingested, and a health promoting action can be exhibited. In addition, when the antioxidant is contained in an external preparation for skin, a cosmetic, or a quasi-pharmaceutical product, it is expected to exhibit not only an effect of preventing oxidation of the contained ingredients but also an effect of preventing aging of the skin or the like.

In addition, saclipin B and/or saclipin A has an anti-glycation action, and is also useful as an active ingredient of an anti-glycation agent. For example, saclipin B and/or saclipin A has been confirmed to be useful as an anti-glycation agent that inhibits the glycation reaction of elastin. Saclipin B has a higher activity of inhibiting the glycation reaction of elastin than saclipin A. The anti-glycation agent that inhibits the glycation reaction of elastin preferably contains saclipin B. In addition, saclipin B and/or saclipin A has been confirmed to be particularly useful as an anti-glycation agent that inhibits the glycation reaction of collagen. Saclipin A has a higher activity of inhibiting the glycation reaction of collagen than saclipin B. The anti-glycation agent that inhibits the glycation reaction of collagen preferably contains saclipin A. It is known that a common reaction is involved in at least a part of the glycation reaction of various proteins. It is presumed that saclipin B and/or saclipin A is also useful as an anti-glycation agent that inhibits the glycation reaction of proteins other than elastin and collagen.

The form of the anti-glycation agent is not particularly limited. The form of the anti-glycation agent is suitable for a food and drink and a pharmaceutical. According to such a form, it is possible to suppress glycation of proteins in the blood vessel wall and the skin tissue in a living body in which the anti-glycation agent has been orally ingested, and to prevent hardening of the blood vessel and deterioration of the skin. In addition, when the anti-glycation agent is contained in an external preparation for skin, a cosmetic, or a quasi-pharmaceutical product, an effect of preventing aging of the skin or the like is expected.

The ultraviolet absorber, the antioxidant, or the anti-glycation agent may appropriately contain an ingredient used for an ordinary ultraviolet absorber, an ordinary antioxidant, or an ordinary anti-glycation agent in addition to saclipin B and saclipin A in a quantitative and qualitative range in which the action of the present disclosure is not impaired. The total amount of saclipin B and saclipin A in the ultraviolet absorber, the antioxidant, or the anti-glycation agent is not particularly limited. The total amount of saclipin B and saclipin A can be 0.0001 mass% to 100 mass% when the mass of the entire preparation is 100 mass% from the viewpoint of securing the handleability such as the storability, the ease of application, and the ease of drinking, and the effectiveness.

The dose of the oral preparation in food and drink, pharmaceutical, and the like can be appropriately changed depending on factors such as the age, weight, sex, and condition of the person who ingests the preparation. For example,

the ingestion or administration amount of the oral preparation is, for example, 0.0001 mg to 100 mg per day of a human adult with a body weight of 60 kg, which is converted to a dry mass of saclipin B and saclipin A, but is not limited to this range. If necessary, the ingestion amount can be once per day or can be divided several times, for example, two or three times per day.

The dose of the external preparation for skin, the cosmetic, or the quasi-pharmaceutical product is not particularly limited. Usually, it can be used by applying it in an appropriate amount to the outer skin such as the skin several times a day. Application examples of the external preparation for skin or the cosmetic will be described later.

3. Utilization of compound (part 2)

A second aspect of the present disclosure is an external preparation for skin or a cosmetic containing at least one compound of the compound represented by the above formula (1) and the compound represented by the above formula (2).

When saclipin B and/or saclipin A is used as an external preparation for skin, the external preparation for skin may further contain an excipient, a base, an emulsifier, a solvent, a stabilizer, and the like. The excipient, carrier, and additive are not particularly limited as long as they are usually used and physiologically or pharmaceutically acceptable, and the type and composition thereof can be appropriately changed. Examples of the dosage form include ointments, solutions, sprays, sheets, granules, and powders.

The total amount of saclipin B and saclipin A in the external preparation for skin is not particularly limited. The total amount of saclipin B and saclipin A in the external preparation for skin can be 0.00001 mass% to 10 mass% when the mass of the entire external preparation for skin is 100 mass% from the viewpoint of securing the storability, the ease of application, and the effectiveness.

When saclipin B and/or saclipin A is used as a cosmetic, the cosmetic may further contain an ingredient generally used as a cosmetic ingredient, for example, a surfactant, an oil ingredient, a moisturizing agent, a film-forming agent, a pigment, a fragrance, and the like. Examples of the form of the cosmetic include a lotion, a cream, an emulsion, a gel, an aerosol, an essence, a pack, a cleaning agent, a foundation, a powder, and various makeup agents (lipstick, blusher, etc.).

The total amount of saclipin B and saclipin A in the cosmetic is not particularly limited. The total amount of saclipin B and saclipin A in the cosmetic can be 0.00001 mass% to 10 mass% when the mass of the entire cosmetic is 100 mass% from the viewpoint of securing the storability, the handleability, and the effectiveness.

4. Method for producing compound (part 1)

A third aspect of the present disclosure is a method for producing a compound, including extracting at least one compound of the compound represented by the above formula (1) and the compound represented by the above formula (2) from cyanobacteria.

As an example of the method for producing a compound, a method in which the cyanobacteria are dried and a compound is extracted from the dried cyanobacteria will be described. The drying of the cyanobacteria is an arbitrary treatment, and may be performed, as necessary. Hereinafter, a method for producing a compound, in which Suizenji-nori is used as an example of the cyanobacteria, will be specifically described.

The conditions for drying Suizenji-nori are not particularly limited. The drying method is not particularly limited, and may be either a natural drying method or an artificial drying method. Examples of the natural drying method include air drying, shade drying, and sun drying. Examples of the artificial drying method include heat drying, hot air drying, vacuum drying, and freeze drying. The drying temperature is preferably 10°C or higher and 60°C or lower, more preferably 15°C or higher and 50°C or lower, and still more preferably 20°C or higher and 40°C or lower from the viewpoint of improving the drying efficiency and suppressing the deactivation of the enzyme involved in the synthesis of saclipin B and saclipin A. The drying time can be appropriately determined according to the drying method to be selected, the wet state of Suizenji-nori, and the like. In the case of the natural drying method, for example, it can be set to half a day to several days. In the case of the artificial drying method, for example, it can be set to 1 minute or longer and 48 hours or shorter. As the dried product of Suizenji-nori, a commercially available dried product of Suizenji-nori may be used.

By drying Suizenji-nori, the yields of saclipin B and saclipin A can be increased as compared with the case where Suizenji-nori is kept in a wet state. Although the mechanism is not clear, it is presumed that drying Suizenji-nori in a viable state induces the generation of saclipin B and saclipin A due to the drying stress, which is considered to be one of the reasons. Note that the present disclosure is not limited by this presumed reason.

A method for extracting a compound is not particularly limited. As an example of a method for extracting a compound, a method, in which Suizenji-nori is crushed, an extraction solvent is added thereto, and the mixture is stirred to obtain saclipin B and saclipin A as extraction liquids, will be described. Incidentally, the crushing and stirring are arbitrary treatments, and if a sufficient yield is obtained, for example, Suizenji-nori may be immersed in the extraction solvent and allowed to stand, whereby saclipin B and/or saclipin A may be extracted.

The crushing of Suizenji-nori can be performed using, for example, a mixer, an ultrasonic crushing device, a bead type crushing device, or the like. A plurality of means may be used in combination for crushing Suizenji-nori. For example, in the case of crushing a dried product of Suizenji-nori, the dried product may preferably be crushed by pulverizing it with a mixer or the like and then subjecting the pulverized product to ultrasonic treatment in an extraction solvent. In the case of crushing Suizenji-nori, which is encapsulated in a gelatinous agar matrix and forms colonies, as it is, Suizenji-nori in a wet state may be homogenized with a mixer or the like, and then subjected to ultrasonic treatment in an extraction solvent to be crushed. The frequency of the ultrasonic treatment can be, for example, 15 kHz to 50 kHz. The temperature of the ultrasonic treatment can be, for example, 4°C or higher and 40°C or lower. The time of the ultrasonic treatment can be, for example, from 5 minutes to 60 minutes.

As the extraction solvent, for example, alcohols such as methanol, ethanol, propanol, and butanol; glycols such as 1,3-butylene glycol, glycerin, and propylene glycol; esters such as ethyl acetate and butyl acetate; ethers such as ethyl ether, propyl ether, isopropyl ether, tetrahydrofuran, and dioxane; halogenated hydrocarbons such as dichloromethane and chloroform; ketones such as acetone, methyl ethyl ketone, diethyl ketone, and cyclohexanone; hexane; cyclohexane; petroleum ether; water, and the like can be used. These solvents can be used alone or as a mixture of two or more kinds thereof.

The extraction conditions are not particularly limited. The extraction temperature can be, for example, 4°C or higher and 40°C or lower. The extraction time can be, for example, 5 minutes or longer and 6 hours or shorter. The amount of the extraction solvent to be added can be 10 mL or more and 100 mL or less with respect to 10 g of the dry weight of Suizenji-nori.

The extraction liquid can be obtained by performing, for example, centrifugation, filtration, suction, compression, and the like to be separated from the residue by solid-liquid separation. The saclipin B and saclipin A obtained as the extraction liquid may be used as they are, or may be used as an extract or a powder by appropriately concentrating or removing the solvent. Furthermore, if necessary, purification can be performed by using one or a combination of two or more appropriate separation and purification means such as chromatography.

5. Method for producing compound (part 2)

A fourth aspect of the present disclosure is a method for producing a compound, in which a solution of the compound represented by the above formula (2) is irradiated with light to obtain the compound represented by the above formula (1).

By irradiating the solution of saclipin A with light, saclipin A can be isomerized to saclipin B. The solvent of the solution of saclipin A is not particularly limited. Examples of the solvent include methanol, ethanol, and acetonitrile. As the solvent, the above-described extraction solvent may be used. Further, the extraction liquid described above may be used as a solution of saclipin A. That is, in the method for producing the present compound, saclipin A derived from Suizenji-nori may be irradiated with light.

The light irradiation conditions are not particularly limited. The illuminance of the light irradiation can be, for example, 4000 lux or more and 10000 lux or less. The temperature at the time of light irradiation can be, for example, 4°C or higher and 40°C or lower. The light irradiation time can be, for example, 12 hours or longer and 48 hours or shorter. The isomerization rate by light irradiation is not particularly limited. The isomerization rate is usually 92% or less, and can be appropriately adjusted, for example, in the range of 10% or more and 92% to obtain a desired ratio of saclipin A and saclipin B.

6. Derivative compound

A fifth aspect of the present disclosure is at least one compound selected from the group consisting of a derivative of the compound represented by the above formula (1) and a derivative of the compound represented by the above formula (2). In the following description, the derivative of the compound represented by the above formula (1) is also referred to as a saclipin B derivative, and the derivative of the compound represented by the above formula (2) is also referred to as a saclipin A derivative.

In the present disclosure, the saclipin B derivative is a derivative obtained by modifying one or more functional groups present in saclipin B, and means a derivative having functionality equivalent to or higher than that of saclipin B. The saclipin A derivative is a derivative obtained by modifying one or more functional groups present in saclipin A, and means a derivative having functionality equivalent to or higher than that of saclipin A. The "functionality" is not particularly limited, and examples thereof include ultraviolet absorbability, antioxidant property, and anti-glycation property.

The saclipin B derivative is preferably one or more selected from the group consisting of an ester derivative obtained by reacting a carboxyl group present at the terminal of saclipin B with an alcohol, an amide derivative obtained by reacting a carboxyl group present at the terminal of saclipin B with an amine, and an ammonium salt obtained by reacting a carboxyl group present at the terminal of saclipin B with ammonium. The saclipin A derivative is preferably one or more

selected from the group consisting of an ester derivative obtained by reacting a carboxyl group present at the terminal of saclipin A with an alcohol, an amide derivative obtained by reacting a carboxyl group present at the terminal of saclipin A with an amine, and an ammonium salt obtained by reacting a carboxyl group present at the terminal of saclipin A with ammonium. The type of the carboxyl group derivative group in the saclipin B derivative and/or the saclipin A derivative can be appropriately selected according to the use, the dosage form when used, and the like.

The carboxyl group derivative group can be represented as, for example, $-COOR^1$, $-CONH_2$, $-CONHR^2$, $-CONR^3R^4$, $-COONH_4$, or $-COONH_3R^5$. Here, $R^1$, $R^2$, $R^3$, $R^4$, and $R^5$ are each independently a hydrocarbon group having 1 to 10 carbon atoms which may have a functional group. Among carboxyl group derivative groups, saclipin B having $-COOCH_3$ and saclipin A having $-COOCH_3$ are also referred to as methyl-esterified saclipin B and methyl-esterified saclipin A. Methyl-esterified saclipin B and methyl-esterified saclipin A can enhance hydrophobicity while securing ultraviolet absorbability equivalent to that of saclipin B and saclipin A. Such a saclipin B derivative and/or saclipin A derivative is more suitable for use as a mixture with a hydrophobic ingredient such as an oil ingredient than saclipin B and/or saclipin A.

The fifth aspect of the present disclosure includes an ultraviolet absorber, an antioxidant, or an anti-glycation agent containing the saclipin B derivative and/or the saclipin A derivative as an active ingredient. In the description of the ultraviolet absorber, antioxidant, or anti-glycation agent of the fifth aspect, "saclipin A" and "saclipin B" in the above "2. Utilization of compound (part 1)" are replaced with "saclipin A derivative" and "saclipin B derivative", respectively, and applied as they are.

The fifth aspect of the present disclosure includes an external preparation for skin or a cosmetic containing the saclipin B derivative and/or the saclipin A derivative. In the description of the external preparation for skin or cosmetic of the fifth aspect, "saclipin A" and "saclipin B" in the above "3. Utilization of compound (part 2)" are replaced with "saclipin A derivative" and "saclipin B derivative", respectively, and applied as they are.

[Examples]

Hereinafter, the present disclosure will be described more specifically by way of examples. However, the scope of the present disclosure is not limited to these examples.

1. Extraction and isolation of compound

Extraction and isolation of the compound from Suizenji-nori were carried out according to the following.

That is, 10 g of dry Suizenji-nori (Product name: "Jusentai", distributor: "Endo Kanagawado", postal code: 838-0031, 2949 Yanaga, Asakura City, Fukuoka Prefecture) was ground with Crush Millser IFM-C20G (Iwatani Corporation), the resulting powder was transferred to a 50 ml tube, then 30 ml of methanol was added and subjected to ultrasonic treatment using an ultrasonic treatment apparatus Model UR-200P (Tomy Seiko Co., Ltd.). The resulting suspension was centrifuged at 2330 ×g for 15 min at 4°C and the resulting supernatant was transferred to another new tube. 20 ml of methanol was added to the remaining pellet, and the mixture was subjected to ultrasonic treatment and centrifugation in the same manner as described above, and the obtained supernatant was also added. The supernatant was then dried in a centrifugal concentrator VC-36R (TAITEC Corporation) under light-shielding conditions. The dried material was dissolved in 5 ml of 55% (v/v) acetonitrile containing 0.1% (v/v) formic acid and then passed through a filter with a pore size of 0.22 μm. This sample was subjected to preparative HPLC with an Inertsil ODS-3 column (particle size 10 μm, inner diameter 50 mm × 20 mm, GL Sciences Inc.) and an Inertsil ODS-3 column (particle size 10 μm, inner diameter 250 mm × 20 mm, GL Sciences Inc.) connected. The operation was performed using 55% (v/v) acetonitrile containing 0.1% (v/v) formic acid as a mobile phase at a flow rate of 6.0 ml/min. Elution of the compound was monitored at a detection wavelength of 320 nm. In this preparative HPLC, saclipin A and saclipin B were eluted as the same fraction. The fraction containing the target compounds was freeze-dried, and then the dried sample was dissolved in a 50% (v/v) acetonitrile solution and was subjected to preparative HPLC using a YMC carotenoid column (particle diameter 5 μm, internal diameter 250 mm × 10 mm). The operation was performed using 50% (v/v) acetonitrile as a mobile phase at a flow rate of 1.0 ml/min. Elution of the compound was monitored at a detection wavelength of 320 nm. In this preparative HPLC, saclipin A was eluted first, followed by saclipin B. Each fraction was freeze-dried to obtain light-green dried samples of saclipin A and saclipin B. From 10 g of a sample of dried Suizenji-nori, 7.0 mg to 10 mg and 1.5 mg to 3.0 mg of saclipin A and saclipin B were obtained, respectively. The content of saclipin A in the dried Suizenji-nori was higher than that of saclipin B.

2. Structure determination of saclipin A and saclipin B

The obtained dried sample was dissolved in methanol, and subjected to absorption spectrum analysis, high-resolution mass spectrometry, and measurement of $^1H$ and $^{13}C$-NMR spectra. The absorption spectra of saclipin A and saclipin B are shown in Fig. 3. In each graph of Fig. 3, the horizontal axis represents wavelength (nm), and the vertical axis

represents relative absorbance.

(1) Saclipin A

[Physicochemical data]

Absorption maximum (methanol solution): 316 nm
Molar absorption coefficient: 26,454 $M^{-1}cm^{-1}$
Precision ESI MS data: calcd for $C_{18}H_{25}O_4$ ($[M-H]^-$): m/z 305.1758; Found: m/z 305.1758
Fragment ions of precision ESI MS: m/z 287.1654, 249.1497, 135.0815, 125.0971
$^1$H-NMR and $^{13}$C-NMR ($CD_3OD$):

[Table 1]

| Position | δ13C | δ1H | mult. *J* Hz |
|---|---|---|---|
| 1 | 181.6 | | |
| 2 | 38.1 | 2.18 | t (7.5) |
| 3 | 27.7 | 1.59 | m |
| 4 | 30.2/30.3/30.7 | 1.38 | m |
| 5 | 30.2/30.3/30.7 | 1.38 | m |
| 6 | 30.2/30.3/30.7 | 1.38 | m |
| 7 | 25.3 | 1.59 | m |
| 8 | 41.8 | 2.68 | t (7.5) |
| 9 | 203.7 | | |
| 10 | 133.8 | 6.38 | d (7.5) |
| 11 | 137.0 | 7.84 | m |
| 12 | 136.3 | 6.54 | m |
| 13 | 136.3 | 6.54 | m |
| 14 | 137.0 | 7.84 | m |
| 15 | 133.6 | 6.35 | d (15) |
| 16 | 203.4 | | |
| 17 | 41.7 | 2.73 | q (7.5) |
| 18 | 8.4 | 1.09 | t (7.5) |

[Two-dimensional NMR]

As a result of heteronuclear multiple-bond correlation spectroscopy (HMBC) analysis, a correlation of HMBC between protons and carbon indicated by arrows in Fig. 4 was observed. In addition, as a result of Nuclear Overhauser Effect Spectroscopy (NOESY) analysis, a NOE correlation indicated by broken lines in Fig. 4 was observed.
From the above physicochemical data, the chemical structure of saclipin A obtained from the dried Suizenji-nori was found.

(2) Saclipin B

[Physicochemical data]

Absorption maximum (methanol solution): 319 nm
Molar absorption coefficient: 30,555 $M^{-1}cm^{-1}$
Precision ESI MS data: calcd for $C_{18}H_{25}O_4$ ($[M-H]^-$): m/z 305.1758; Found: m/z 305.1757
Fragment ions of precision ESI MS: m/z 287.1655, 249.1496, 135.0815, 125.0971

$^1H$-NMR and $^{13}C$-NMR ($CD_3OD$):

[Table 2]

| Position | δ13C | δ1H | Mult. *J* Hz |
|---|---|---|---|
| 1 | 183.3 | | |
| 2 | 39.1 | 2.14 | t (7.5) |
| 3 | 27.8 | 1.59 | m |
| 4 | 30.3/30.4/30.7 | 1.36 | m |
| 5 | 30.3/30.4/30.7 | 1.36 | m |
| 6 | 30.3/30.4/30.7 | 1.36 | m |
| 7 | 25.5 | 1.59 | m |
| 8 | 42.9 | 2.63 | t (7.5) |
| 9 | 203.4/203.2 | | |
| 10 | 133.4/133.1 | 6.36 | d (15) |
| 11 | 142.5 | 7.32 | AA'BB' type |
| 12 | 133.9 | 6.84 | AA'BB' type |
| 13 | 139.9 | 6.84 | AA'BB' type |
| 14 | 142.5 | 7.32 | AA'BB' type |
| 15 | 133.4/133.1 | 6.36 | d (15) |
| 16 | 203.4/203.2 | | |
| 17 | 41.7 | 2.67 | q (7.5) |
| 18 | 8.4 | 1.080 | t (7.5) |

As a result of heteronuclear multiple-bond correlation spectroscopy (HMBC) analysis, a correlation of HMBC between protons and carbon indicated by arrows in Fig. 5 was observed. In addition, as a result of Nuclear Overhauser Effect Spectroscopy (NOESY) analysis, a NOE correlation indicated by broken lines in Fig. 5 was observed.

From the above physicochemical data, the chemical structure of saclipin B obtained from the dried Suizenji-nori was found.

3. Induction of saclipin A and saclipin B by drying treatment

Saclipin A and saclipin B can be induced by subjecting raw Suizenji-nori to a drying treatment.

50 g of fresh Suizenji-nori harvested from the Kogane River was mixed with 10 ml of river water and homogenized in Crush Millser IFM-C20G (Iwatani Corporation). The homogenized sample was placed in plastic trays in portions of 5.0 g and dried by a drying oven DY400 (Yamato Scientific Co., Ltd.) set at 30°C under dark conditions. A control experiment was also conducted in which the sample was sealed in a plastic bag and kept moist. Water was added to the sample subjected to the drying treatment to match the weight before the drying treatment, and then methanol was added so as to have a final concentration of 80% (v/v). Thereafter, sufficient ultrasonic treatment was performed using an ultrasonic treatment apparatus Model UR-200P (Tomy Seiko Co., Ltd.) to extract saclipin. The sample subjected to the ultrasonic treatment was centrifuged at 2330 ×g for 15 minutes at 4°C, and then the obtained supernatant was subjected to HPLC analysis to quantify saclipin. The content of saclipin A and saclipin B of the control sample not subjected to the drying treatment was 0.005 mg/g and 0.001 mg/g, respectively, per wet weight, whereas the content contained in the sample was significantly increased to 0.056 mg/g and 0.008 mg/g, respectively, by subjecting the drying treatment. From these results, it was found that saclipin A and saclipin B are substances induced in the drying treatment step of Suizenji-nori.

4. Generation of saclipin B by photoisomerization

Saclipin A purified from dried Suizenji-nori was dissolved in methanol, ethanol or acetonitrile, and the solution was irradiated with light of 6000 lux under a white fluorescent lamp for 24 hours or longer. Thereafter, HPLC analysis was performed under the following measurement conditions to calculate the amounts of saclipin A and saclipin B, and the isomerization rate of saclipin A was calculated. The chromatogram of the saclipin A solution before light irradiation is shown on the left in Fig. 6, and the chromatogram of the saclipin A solution after light irradiation is shown on the right in Fig. 6.

[Measurement conditions]

Detection wavelength: 320 nm
Column: Inerteil ODS-P 3 μm, 4.6×33 + 4.6×150 mm
Eluent: 50% Acetonitrile (isocratic)

About 90% of the dissolved saclipin A could be converted to saclipin B. The amount ratio of saclipin A and saclipin B did not change for at least 96 hours even after dark treatment after light irradiation. From this result, it was found that saclipin B was generated from saclipin A by a photoisomerization reaction.

5. Measurement of physiological activity of saclipin A and saclipin B

(1) Test Example 1: evaluation of antioxidant activity

(1.1) Measurement of ABTS radical scavenging activity

To evaluate the antioxidant activity of saclipin A and saclipin B, 2,2-azino-bis(3-ethylbenzothiazoline-6-sulfonic acid) diammonium salt (ABTS) radical scavenging activity was measured.
A 7 mM ABTS aqueous solution and a 2.45 mM potassium persulfate aqueous solution were mixed at 25°C for 16 hours in the dark to prepare an ABTS radical solution. The ABTS radical solution was diluted with ethanol such that the absorbance at 734 nm was about 1.0. 50 μL of this diluted ABTS radical solution and 50 μL of the test sample ethanol solution were mixed, and the mixed reactant was held at room temperature for 20 or 40 minutes in the dark, then the absorbance was measured at 734 nm. The final reaction concentration of the sample was adjusted to 0 mM, 0.06 mM, 0.12 mM, 0.18 mM, 0.24 mM, 0.30 mM, 0.60 mM, 1.20 mM, and 1.80 mM. Ascorbic acid and Trolox were used as standard samples. Inhibition rates were calculated using the following equation.

$$\text{Inhibition activity (\%)} = ((Ab - As)/Ab) \times 100$$

Here, Ab and As represent the absorbances of the blank sample and the test sample, respectively. The 50% inhibition concentration ($IC_{50}$) values calculated from the measurement results are shown in Table 3.
The measurement results of the antioxidant activity are as follows.

[Table 3]

| Measurement of radical scavenging activity by ABTS method | | |
|---|---|---|
| Compound | $IC_{50}$ value (μM) | |
| | 20 min | 40 min |
| Saclipin A | 974 | 717 |
| Saclipin B | 182 | 142 |
| Ascorbic acid | 7.9 | 7.5 |
| Trolox | 6.7 | 6.7 |

From the $IC_{50}$ values shown in Table 3, it was confirmed that both saclipin A and saclipin B showed radical scavenging activity against ABTS radicals. Saclipin B was found to be more active than saclipin A.

(1.2) Evaluation of temporal change in antioxidant activity of saclipin

From the experimental results in Table 3, it was suggested that saclipin was an antioxidant that gradually functioned because the scavenging rate of ABTS radicals by saclipin increased at the lapse of the reaction time of 40 minutes as compared with at the lapse of the reaction time of 20 minutes. Therefore, the temporal change of radical scavenging was measured by a time course experiment. In the time course experiment, the decrease in absorbance after addition of the test sample was recorded continuously for 2900 seconds at 25°C and 734 nm. In the time course experiment, the final reaction concentrations of saclipin A, saclipin B, ascorbic acid, and Trolox were adjusted to 0.60 mM,

0.18 mM, 6 μM, and 6 μM, respectively.

The measurement results of the time course experiment are as follows.

It was found that the radical scavenging reaction of both saclipin A and saclipin B proceeded even after 2900 seconds from the addition to the reaction system (Figs. 7(A) and 7(B)). The ascorbic acid and Trolox of the antioxidant used as a standard sample hardly functioned after a radical scavenging reaction occurred immediately after addition (Figs. 7(C) and 7(D)). From these results, it was found that both saclipin A and saclipin B have a radical scavenging action and are slow-acting antioxidant substances.

(2) Test Example 2: evaluation of anti-glycation activity

To evaluate the anti-glycation activity of saclipin A and saclipin B, the effect on glycation of elastin and collagen was investigated.

The effect of saclipin on the glycation of elastin and collagen was evaluated using an elastin anti-glycation assay kit (product number: AAS-AGE-K05, Cosmo Bio Co., Ltd.) and a collagen anti-glycation assay kit (product number: AK71, Cosmo Bio Co., Ltd.), respectively. The assay was performed according to the manufacturer's protocol using saclipin A and saclipin B purified from Suizenji-nori. However, since saclipin was not dissolved in the attached aqueous buffer solution, it was dissolved in dimethyl sulfoxide (DMSO) and subjected to an experiment. The final reaction concentration of the sample was adjusted to 0 mM, 0.5 mM, 1.0 mM, 2.0 mM, and 4.0 mM. The measurement results are shown in Figs. 8 and 9.

The measurement results of the anti-glycation activity are as follows.

As an inhibition effect on the glycation reaction of elastin, the $IC_{50}$ value of saclipin A was 2.5 mM. Since it was comparable to the $IC_{50}$ value (1.5 mM) of aminoguanidine which is a known glycation inhibitor, it was found that saclipin A inhibited the glycation reaction of elastin (Fig. 8). The activity of saclipin B was slightly weaker than the activity of saclipin A, but about 45% of the glycation reaction was inhibited at a concentration of 4 mM. On the other hand, as shown in Fig. 9, saclipin exhibited a stronger inhibition effect on glycation of collagen. The $IC_{50}$ value for aminoguanidine was 2.4 mM, whereas the $IC_{50}$ values for saclipin A and saclipin B were 1.9 mM and 0.9 mM, respectively. From these results, it became clear that both saclipin A and saclipin B have functions as glycation inhibitors of elastin and collagen which are components of the dermis.

6. Generation of saclipin A derivative and saclipin B derivative

Saclipin A derivative and saclipin B derivative were generated using a fatty acid methylation kit (product No. 06482-04, Nacalai Tesque, Inc.). The assay was performed according to the manufacturer's protocol using saclipin A and saclipin B purified. However, reagent B was not used, and only reagent C was used. The reagent C is a reagent for methylating free fatty acid by reacting free fatty acid with methanol in the presence of an acid catalyst. The extraction reagent was added and mixed, and then the upper layer was fractionated from the separated two layers.

The ingredients obtained by fractionating the upper layer were subjected to reverse phase HPLC analysis. Saclipin A and saclipin B were also subjected to reverse phase HPLC analysis under the same conditions. The results are shown in Fig. 10. The upper part of Fig. 10 is a chromatogram of saclipin A and saclipin B, and the lower part is a chromatogram of the upper layer ingredient. In the chromatogram in the upper part, a peak "Sac A" at a retention time of 12 min to 15 min is a peak of saclipin A, and a peak "Sac B" at a retention time of 15 min to 18 min is a peak of saclipin B. Methyl esterification of saclipin A and saclipin B increases hydrophobicity and increases the retention time in reverse phase HPLC analysis. In the chromatogram of the upper layer ingredient in the lower part, peaks were confirmed at a retention time of 40 min to 50 min and a retention time of 60 min to 70 min. From this, it was found that saclipin A and saclipin B were methyl-esterified. "Sac A_methyl ester" and "Sac B_methyl ester" are attached to the respective peaks.

The absorption spectra of "saclipin A (Sac A)", "saclipin B (Sac B)", "methyl-esterified saclipin A (Sac A_methyl ester)", and "methyl-esterified saclipin B (Sac B_methyl ester)" were acquired by the method described in "2. Structure determination of saclipin A and saclipin B" described above. The results are shown in Fig. 11. In each graph of Fig. 11, the horizontal axis represents wavelength (nm), and the vertical axis represents relative absorbance. The maximum point of saclipin A was 317 nm. The maximum point of methyl-esterified saclipin A was 316 nm. The maximum point of saclipin B was 319 nm. The maximum point of methyl-esterified saclipin B was 319 nm.

From these results, it was found that the methyl-esterified saclipin A has ultraviolet absorbability equivalent to that of saclipin A. In addition, it was found that the methyl-esterified saclipin B has ultraviolet absorbability equivalent to that of saclipin B.

It is presumed that the functionality of saclipin A and saclipin B is mainly attributed to the regions containing carbon-carbon double bonds. In methyl-esterified saclipin A and methyl-esterified saclipin B, these regions containing carbon-carbon double bonds remain unchanged. Therefore, it is a reasonable result that the methyl-esterified saclipin A and the methyl-esterified saclipin B could retain ultraviolet absorbability equivalent to that of saclipin A and saclipin B. That

is, it is presumed that the saclipin A derivative and the saclipin B derivative can retain functionality equivalent to that of saclipin A and saclipin B, not only in ultraviolet absorbability but also in other functionalities, as long as they have regions containing carbon-carbon double bonds.

7. Biocompatibility test

In order to examine the cytotoxicity of saclipin A and saclipin B, MTT tests were performed on (1) neonatal human dermal fibroblasts and (2) human keratinocytes.

(1) MTT test on neonatal human dermal fibroblasts

The neonatal human dermal fibroblasts (NHDF) were purchased from ATCC. The neonatal human dermal fibroblasts were seeded in 96-well plates at $1\times10^4$ cells/well. Neonatal human dermal fibroblasts were cultured overnight at 37°C, 5% $CO_2$ using Dulbecco's modified eagle medium (DMEM) in which 10% fetal bovine serum, 100 μg/mL penicillin, 100 units/mL streptomycin, and 10 mM HEPES were added to DMEM. 0.1 μM, 0.36 μM, 1.0 μM, 3.6 μM, and 10.0 μM of saclipin A or saclipin B were added to each cell. As a control, 1% ethanol was added to the cells. Next, 0.5 mg/mL MTT(3-(4,5-dimethylthiazole-2-yl)-2,5-diphenyltetrazolium bromide) was added, and the mixture was cultured for 20 hours at 37°C, 5% $CO_2$. The whole formazan crystals were dissolved in dimethyl sulfoxide (DMSO), and then the absorbance of each sample solution was measured at 540 nm to determine the cell viability. The results are shown in Fig. 12.

The upper part of Fig. 12 is a graph showing the results of cell viability (%). The labels on the horizontal axis represent each sample corresponding to the bar graph. For example, the labels "saclipin A" "10.00" represent that the cells were treated with 10.0 μM saclipin A. The vertical axis represents the cell viability when the cell viability of cells treated with 1% ethanol is taken as 100%.

From the graph in the upper part of Fig. 12, the cytotoxicity of saclipin A was not higher than that of 1% ethanol at any concentration. Similarly, the cytotoxicity of saclipin B was not higher than that of 1% ethanol at any concentration.

The lower part of Fig. 12 provides microphotographs of "cells treated with 1% ethanol", "cells treated with 10.0 μM saclipin A", and "cells treated with 10.0 μM saclipin B" from the left.

The "cells treated with 10.0 μM saclipin A" exhibited an appearance similar to that of the "cells treated with 1% ethanol", and no abnormality was observed. It was suggested that saclipin A had high biocompatibility. The "cells treated with 10.0 μM saclipin B" exhibited an appearance similar to that of the "cells treated with 1% ethanol", and no abnormality was observed. It was suggested that saclipin B had high biocompatibility.

(2) MTT test on human keratinocytes

PSVK1 cells (JCRB1093, JCRB Cell Bank, National Institute of Biomedical Innovation, Health and Nutrition, Japan) were used as human keratinocytes. In this test, the test was carried out in the same manner as in "(1) MTT test on neonatal human dermal fibroblasts" described above except that the cells were cultured using a keratinocyte medium (Thermo Fisher Scientific) supplemented with 0.05 mg/mL bovine pituitary extract, 0.005 μg/mL epidermal growth factor, 100 units/mL penicillin, and 100 μg/mL streptomycin (Gibco). The results are shown in Fig. 13.

The upper part of Fig. 13 is a graph showing the results of cell viability (cell viability, %). The labels on the horizontal axis represent each sample corresponding to each bar graph. For example, the labels "saclipin A" "10.00" represent that the cells were treated with 10.0 μM saclipin A. The vertical axis represents the cell viability when the cell viability of cells treated with 1% ethanol is taken as 100%.

From the graph in the upper part of Fig. 13, the cytotoxicity of saclipin A was not higher than that of 1% ethanol at any concentration. Similarly, the cytotoxicity of saclipin B was not higher than that of 1% ethanol at any concentration.

The lower part of Fig. 13 provides microphotographs of "cells treated with 1% ethanol", "cells treated with 10.0 μM saclipin A", and "cells treated with 10.0 μM saclipin B" from the left.

The "cells treated with 10.0 μM saclipin A" exhibited an appearance similar to that of the "cells treated with 1% ethanol", and no abnormality was observed. It was suggested that saclipin A had high biocompatibility. The "cells treated with 10.0 μM saclipin B" exhibited an appearance similar to that of the "cells treated with 1% ethanol", and no abnormality was observed. It was suggested that saclipin B had high biocompatibility.

8. Summary

According to the present example, it was possible to provide a novel ultraviolet absorber, a novel antioxidant, a novel anti-glycation agent, a novel external preparation for skin, and a novel cosmetic. According to the present example, it was possible to provide a novel method for producing a compound. According to the present example, it was possible to

provide a novel compound.

## Claims

1. An ultraviolet absorber, an antioxidant, or an anti-glycation agent comprising, as an active ingredient, at least one compound of a compound represented by the following formula (1) and a compound represented by the following formula (2).

[Chemical Formula 1]

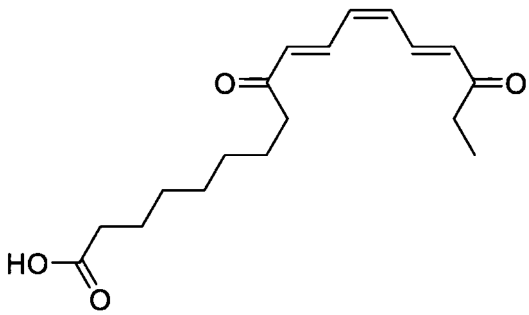

(1)

[Chemical Formula 2]

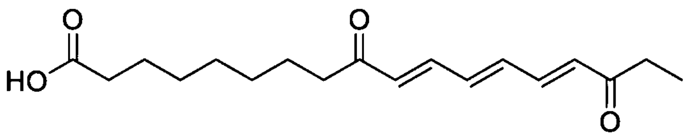

(2)

2. An external preparation for skin or a cosmetic comprising at least one compound of a compound represented by the following formula (1) and a compound represented by the following formula (2).

[Chemical Formula 3]

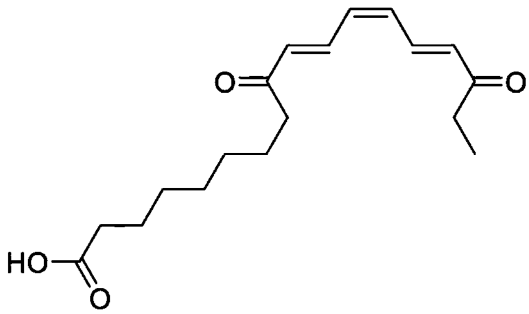

(1)

[Chemical Formula 4]

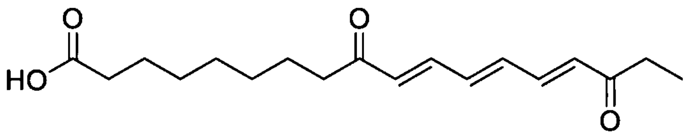

(2)

**3.** A method for producing a compound, comprising extracting at least one compound of a compound represented by the following formula (1) and a compound represented by the following formula (2) from cyanobacteria.

[Chemical Formula 5]

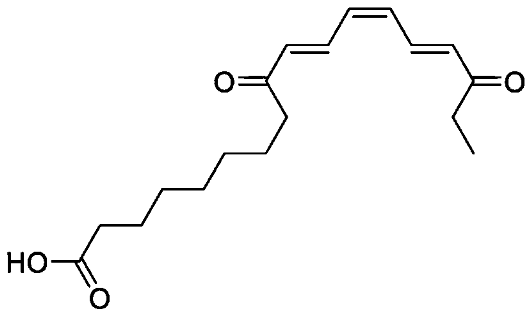

(1)

[Chemical Formula 6]

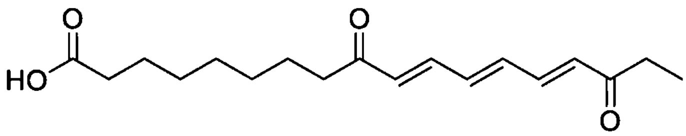

(2)

4. The method for producing a compound according to claim 3, further comprising drying the cyanobacteria before the extracting of the compound.

5. A method for producing a compound, comprising irradiating a solution of a compound represented by the following formula (2) with light to obtain a compound represented by the following formula (1).

[Chemical Formula 7]

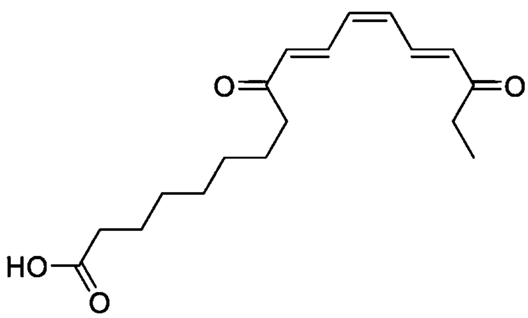

(1)

[Chemical Formula 8]

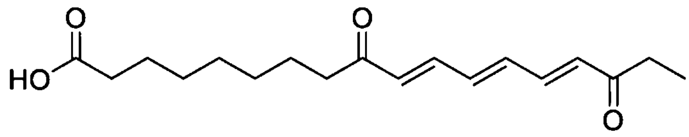

(2)

**6.** At least one compound selected from the group consisting of a derivative of a compound represented by the following formula (1) and a derivative of a compound represented by the following formula (2).

[Chemical Formula 9]

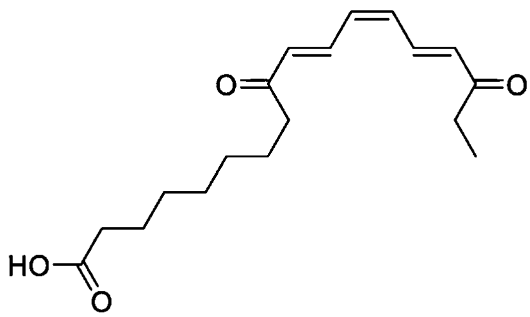

(1)

[Chemical Formula 10]

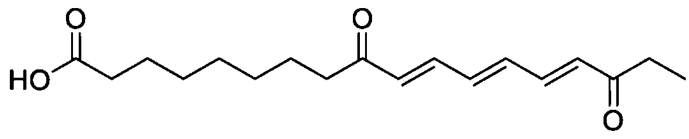

(2)

**7.** An ultraviolet absorber, an antioxidant, or an anti-glycation agent comprising the compound according to claim 6 as an

active ingredient.

**8.** An external preparation for skin or a cosmetic comprising the compound according to claim 6.

Fig. 1

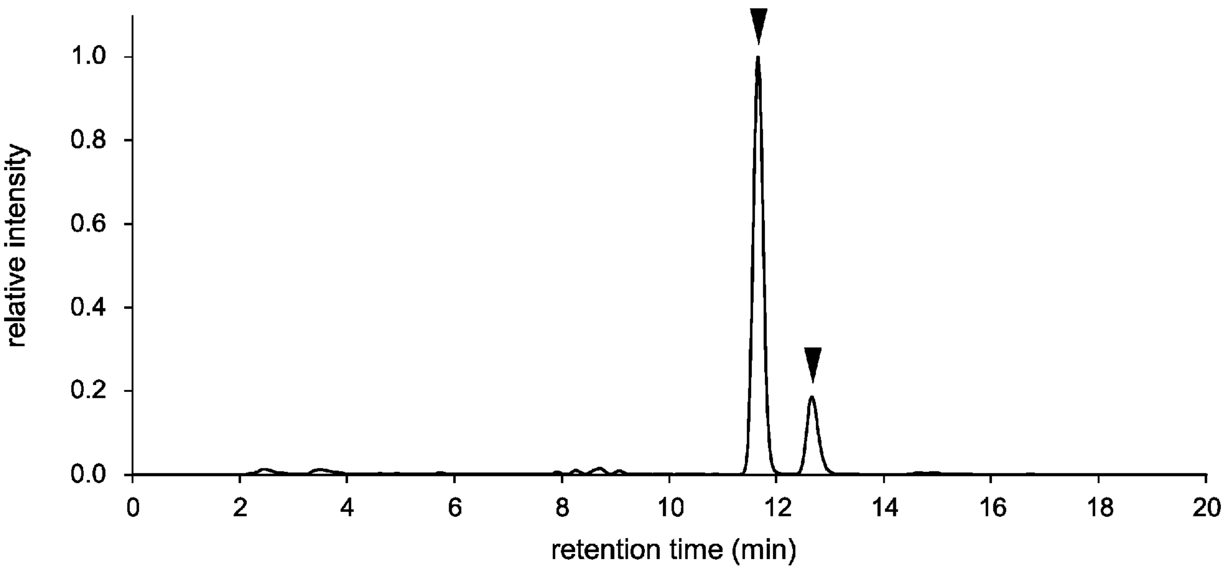
1.0
0.8
0.6
0.4
0.2
0.0
relative intensity
0
2
4
6
8
10
12
14
16
18
20
retention time (min)

Fig. 2

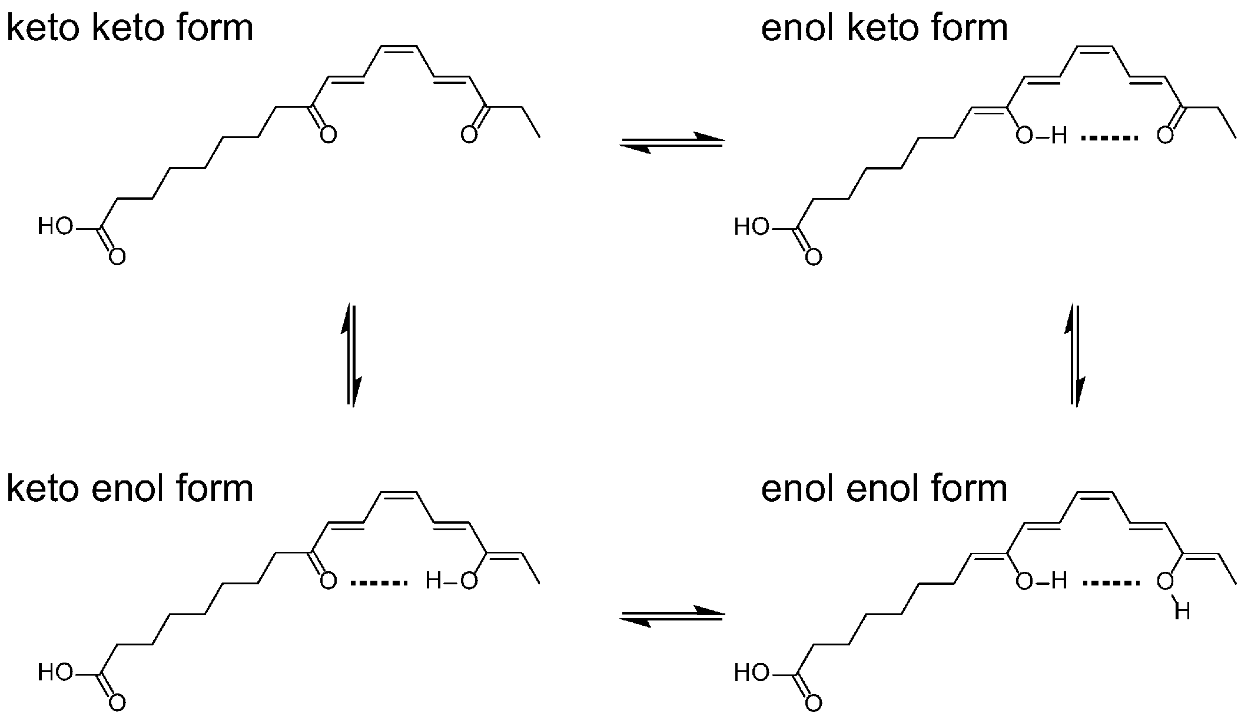

# Fig. 3

| | |
|---|---|
| Saclipin A | 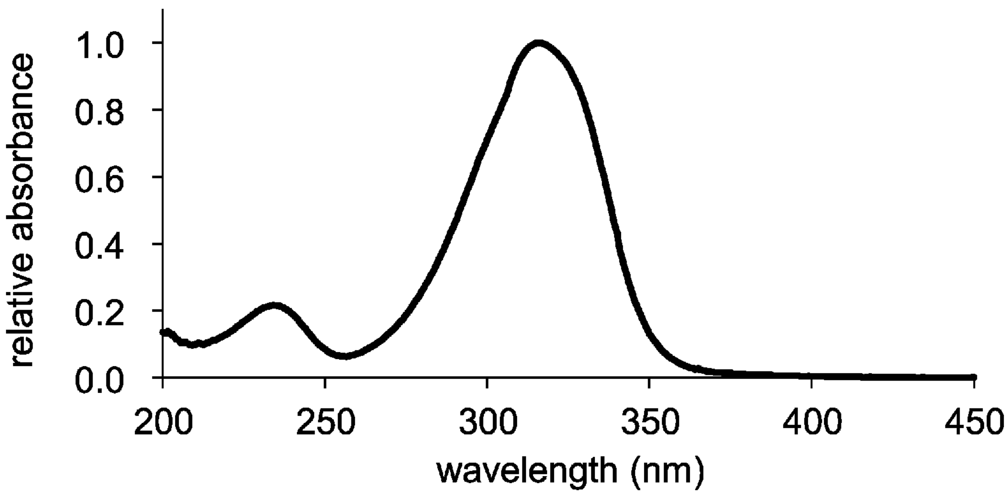  |
| Saclipin B | 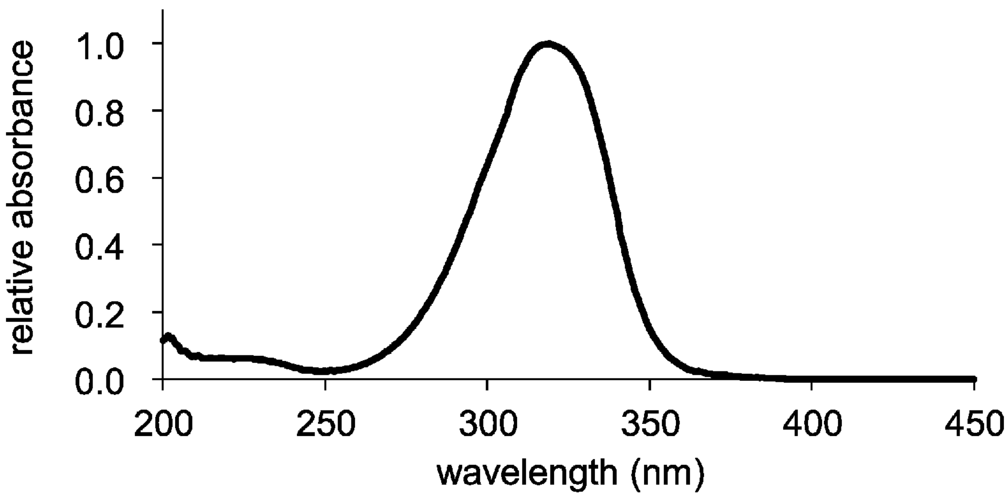  |

Fig. 4

Saclipin A

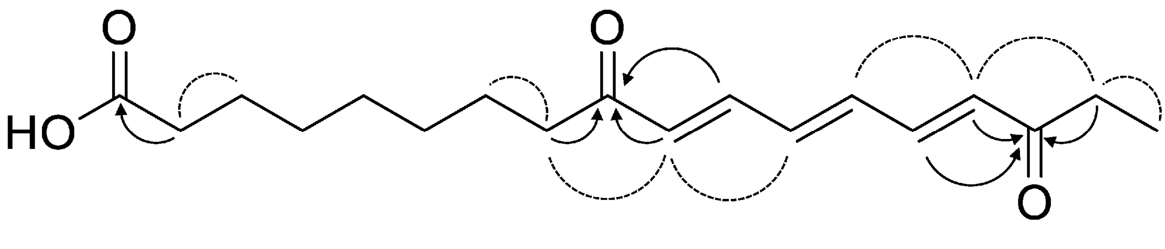

Fig. 5

Saclipin B

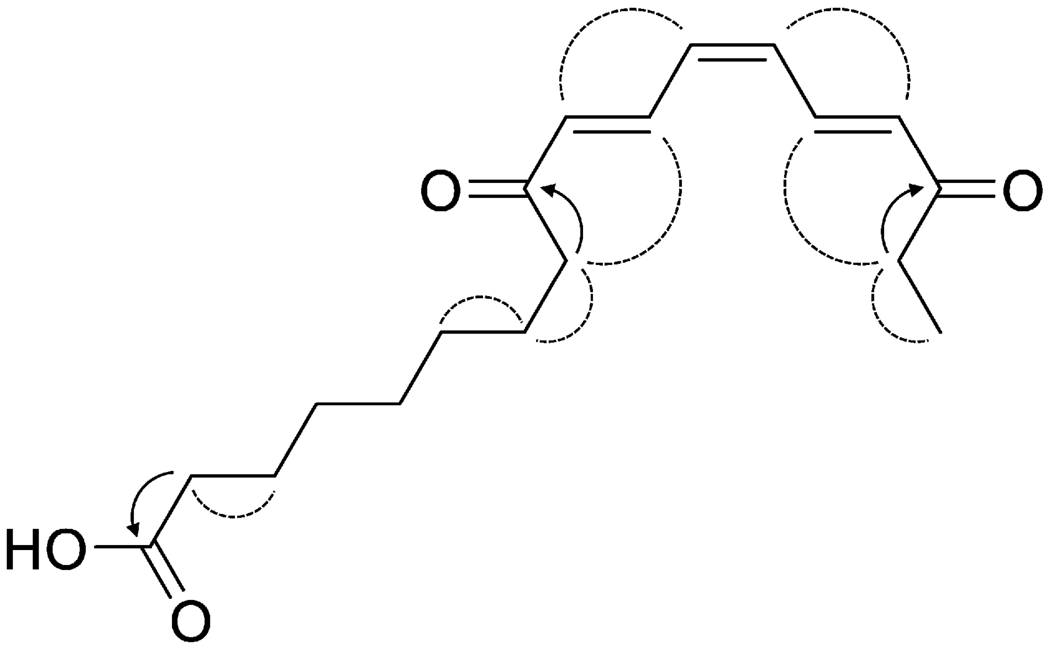

Fig. 6

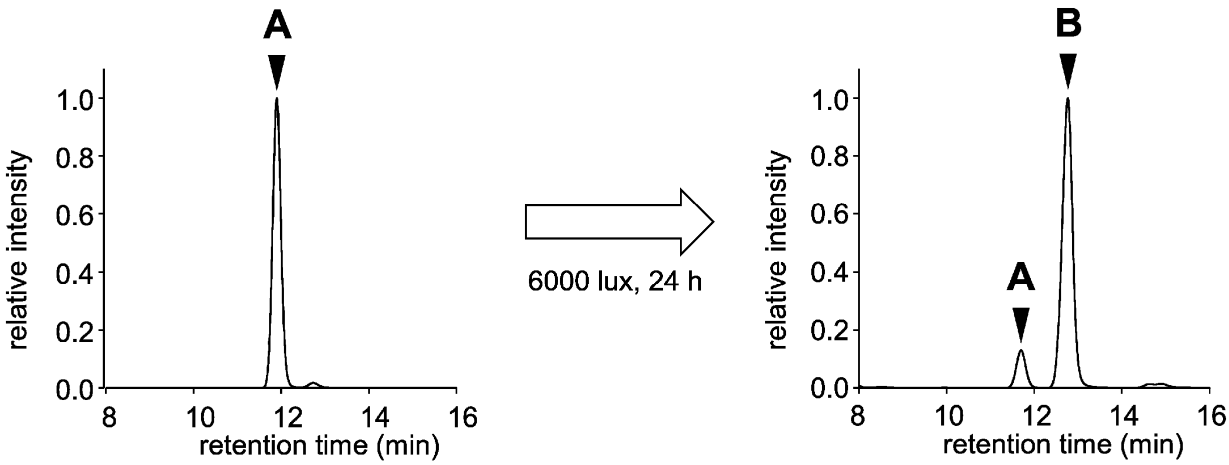
A
relative intensity
1.0
0.8
0.6
0.4
0.2
0.0
8
10
12
14
16
retention time (min)
6000 lux, 24 h
B
A
relative intensity
1.0
0.8
0.6
0.4
0.2
0.0
8
10
12
14
16
retention time (min)

Fig. 7

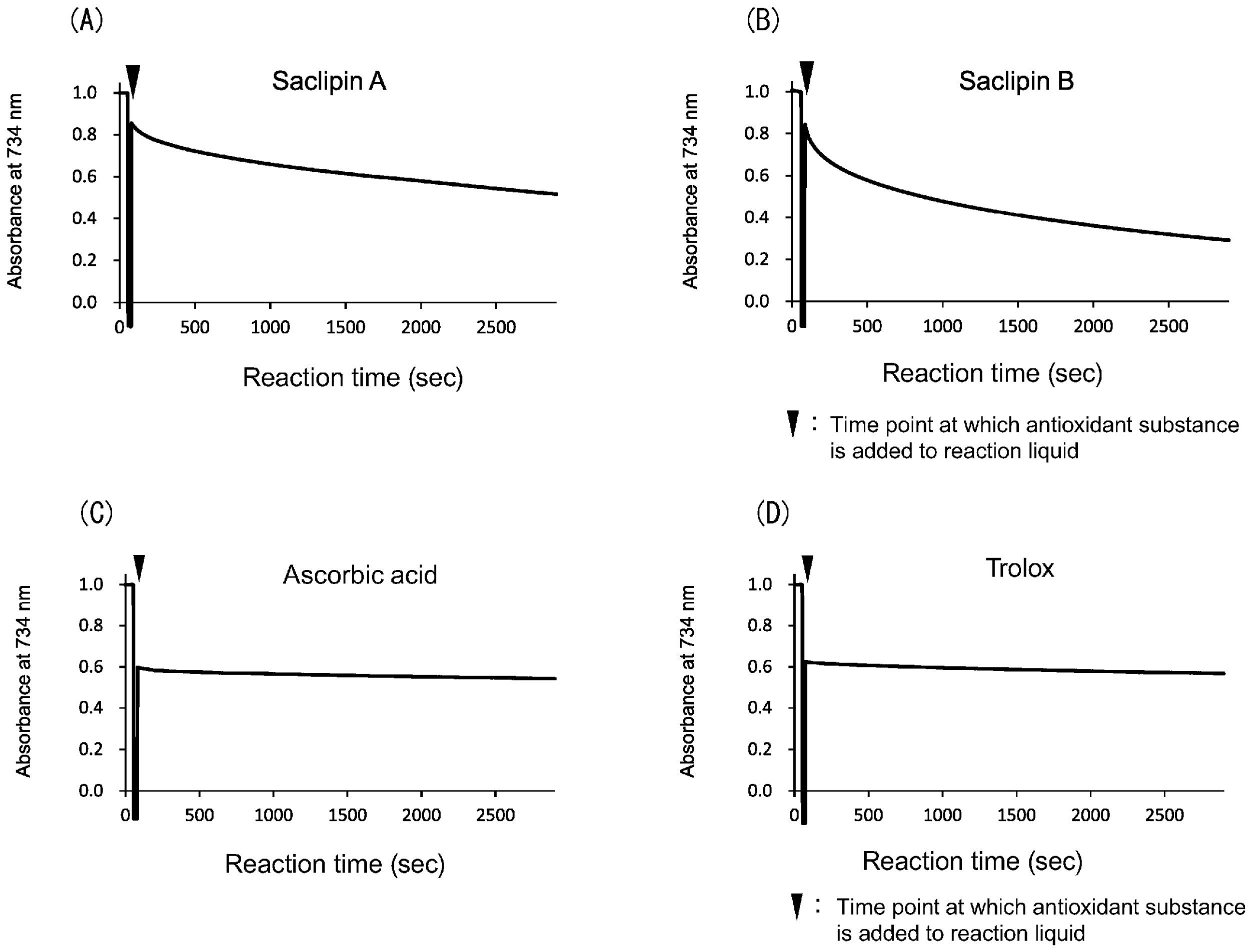
(A)
Saclipin A
Absorbance at 734 nm
1.0
0.8
0.6
0.4
0.2
0.0
0
500
1000
1500
2000
2500
Reaction time (sec)
(B)
Saclipin B
Absorbance at 734 nm
Reaction time (sec)
▼ : Time point at which antioxidant substance is added to reaction liquid
(C)
Ascorbic acid
Absorbance at 734 nm
Reaction time (sec)
(D)
Trolox
Absorbance at 734 nm
Reaction time (sec)
▼ : Time point at which antioxidant substance is added to reaction liquid

Fig. 8

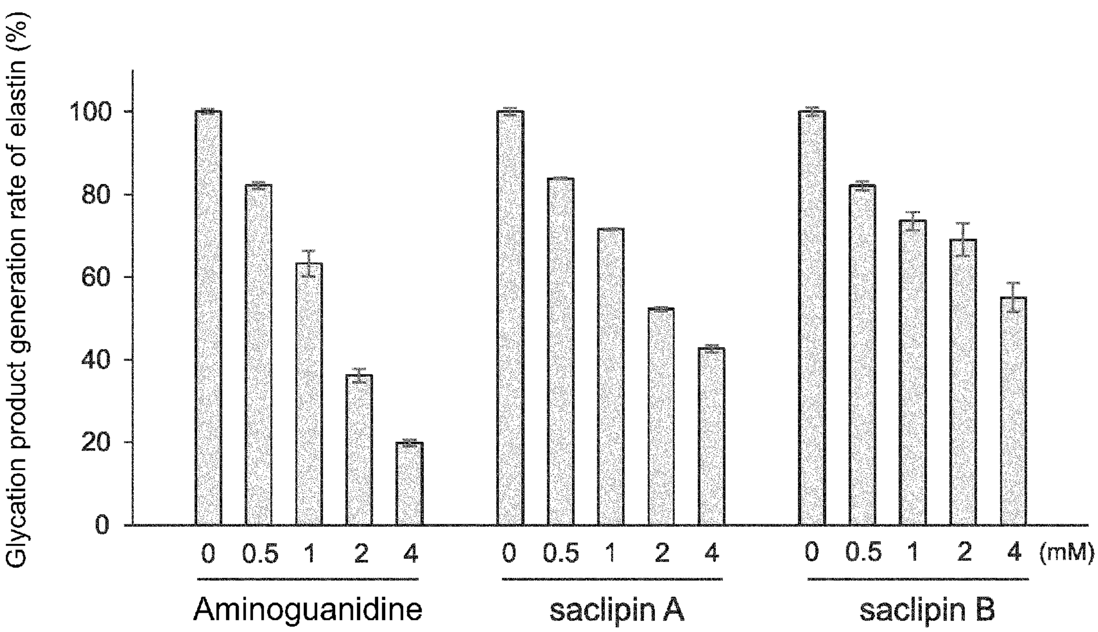
Glycation product generation rate of elastin (%)
100
80
60
40
20
0
0 0.5 1 2 4
0 0.5 1 2 4
0 0.5 1 2 4 (mM)
Aminoguanidine
saclipin A
saclipin B

# Fig. 9

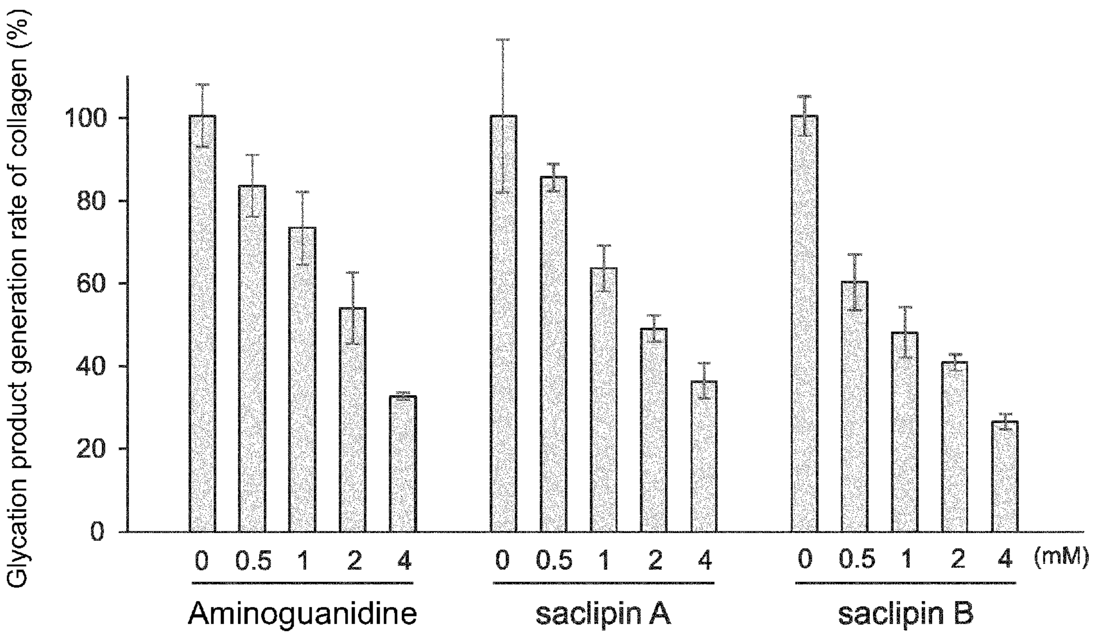
Glycation product generation rate of collagen (%)
0
20
40
60
80
100
0 0.5 1 2 4
0 0.5 1 2 4
0 0.5 1 2 4 (mM)
Aminoguanidine
saclipin A
saclipin B

Fig. 10

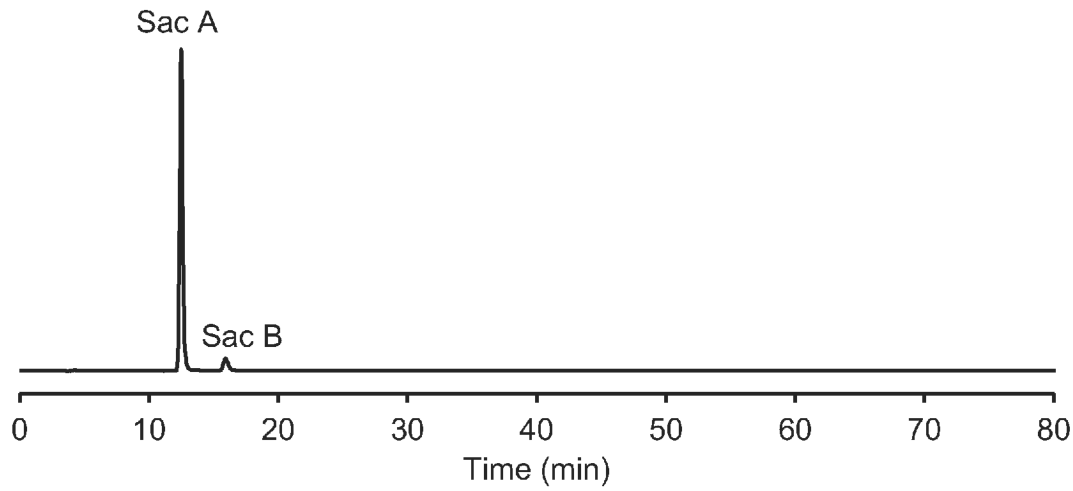
Sac A
Sac B
0
10
20
30
40
50
60
70
80
Time (min)

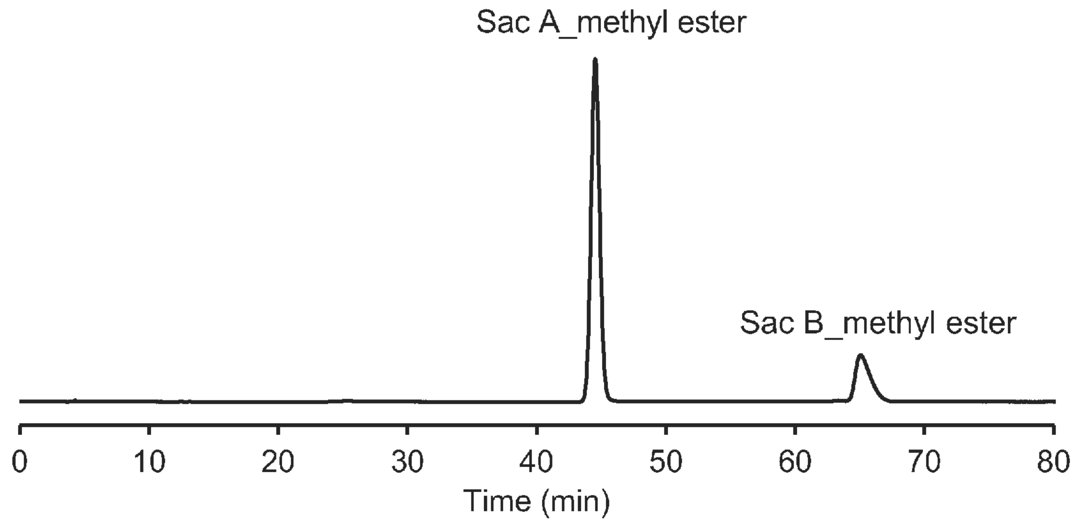
Sac A_methyl ester
Sac B_methyl ester
0
10
20
30
40
50
60
70
80
Time (min)

Fig. 11

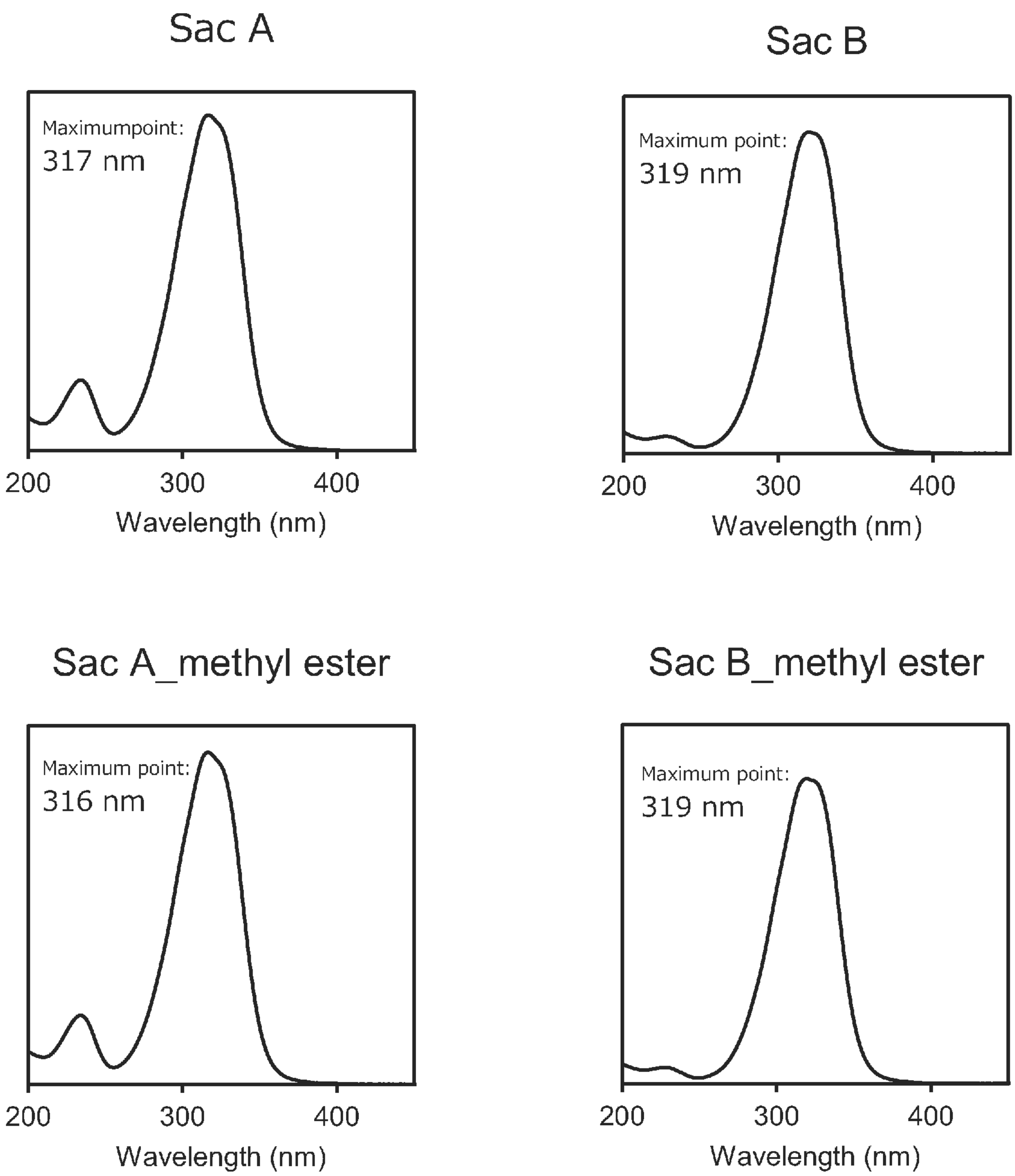
Sac A
Maximumpoint:
317 nm
200
300
400
Wavelength (nm)
Sac B
Maximum point:
319 nm
200
300
400
Wavelength (nm)
Sac A_methyl ester
Maximum point:
316 nm
200
300
400
Wavelength (nm)
Sac B_methyl ester
Maximum point:
319 nm
200
300
400
Wavelength (nm)

Fig. 12

Primary neonatal human dermal fibroblast

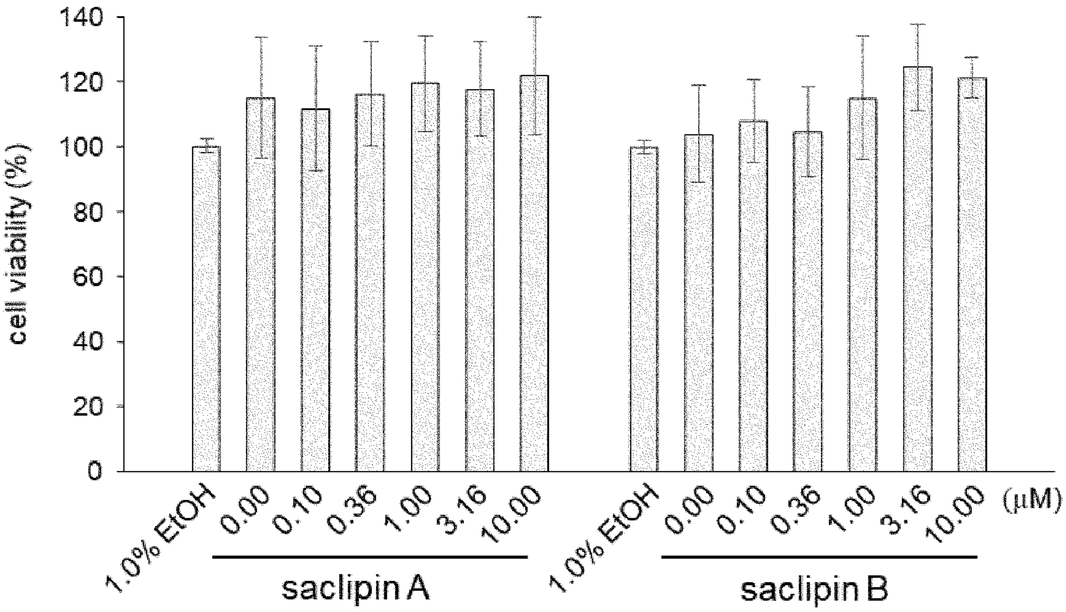


1.0% EtOH | 10 μM saclipin A | 10 μM saclipin B

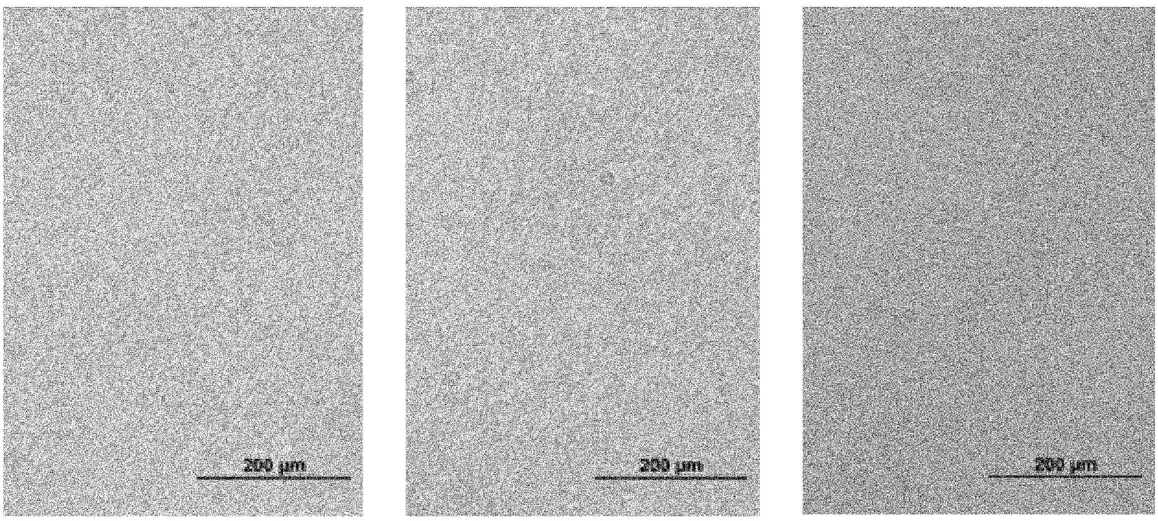

# Fig. 13

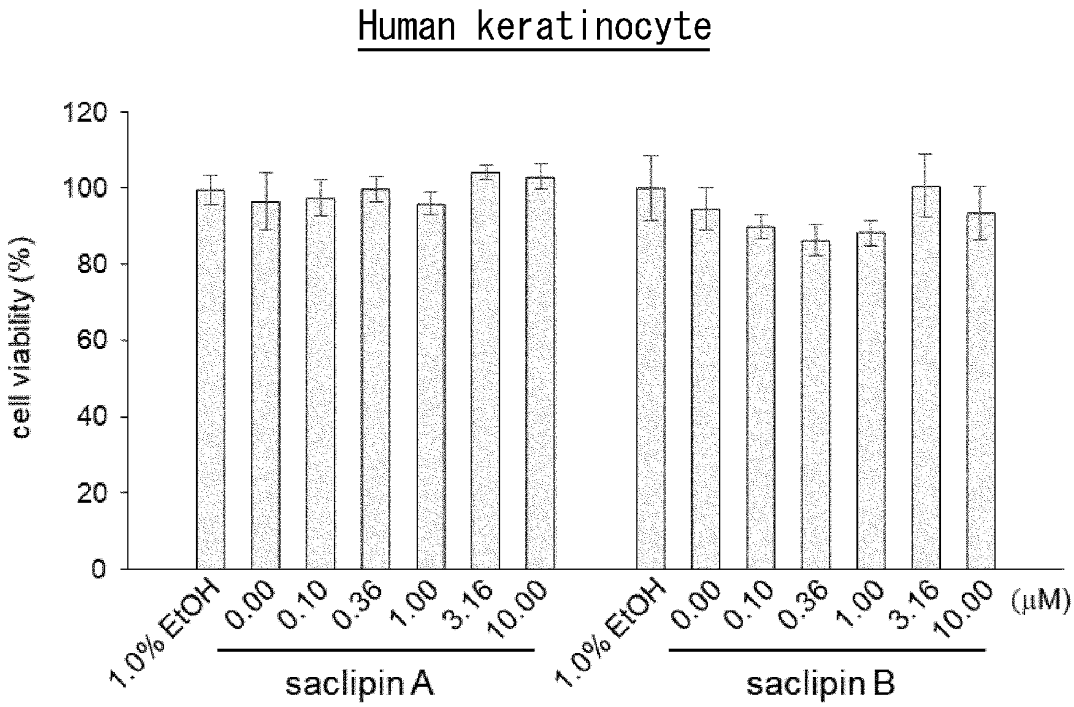


1.0% EtOH

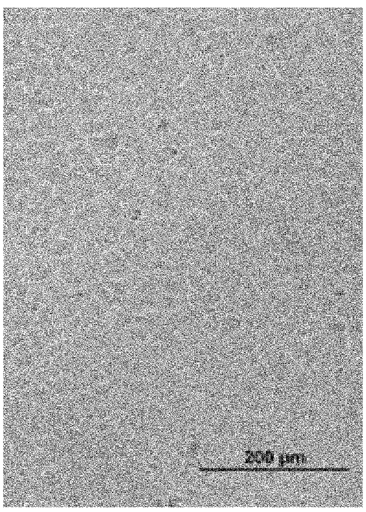


10 μM saclipin A

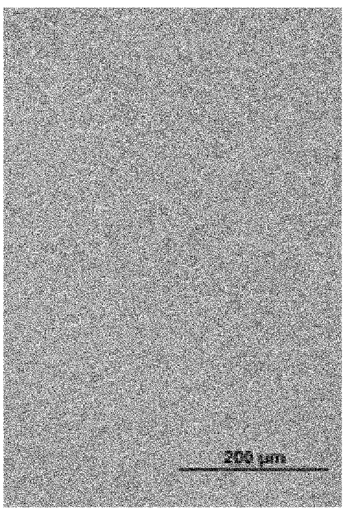


10 μM saclipin B

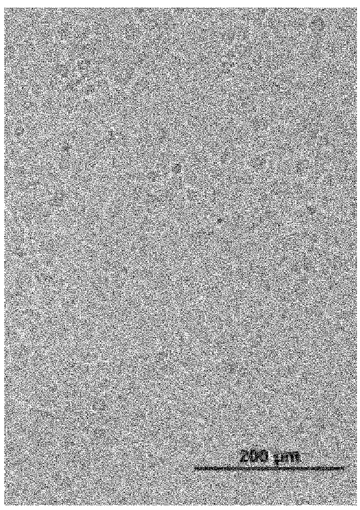

**INTERNATIONAL SEARCH REPORT**

International application No.
**PCT/JP2024/020712**

**A. CLASSIFICATION OF SUBJECT MATTER**

***C09K 3/00***(2006.01)i; ***A23L 33/10***(2016.01)i; ***A23L 33/105***(2016.01)i; ***A61K 8/36***(2006.01)i; ***A61K 31/202***(2006.01)i; ***A61Q 19/00***(2006.01)i; ***C07C 51/47***(2006.01)i; ***C07C 51/353***(2006.01)i; ***C07C 59/76***(2006.01)i; ***C09K 15/06***(2006.01)i
FI: C09K3/00 104; C07C51/47; C07C51/353; C09K15/06; A61K31/202; A61K8/36; A61Q19/00; A23L33/10; A23L33/105; C07C59/76 CSP

According to International Patent Classification (IPC) or to both national classification and IPC

**B. FIELDS SEARCHED**

Minimum documentation searched (classification system followed by classification symbols)

C09K3/00; A23L33/10; A23L33/105; A61K8/36; A61K31/202; A61Q19/00; C07C51/47; C07C51/353; C07C59/76; C09K15/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)JSTPlus/JMEDPlus/JST7580 (JDreamIII)Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | BOWEN, L. et al. Chemical constituents, cytotoxic and antioxidant activities of extract from the rhizomes of Osmunda japonica Thunb. Natural Product Research. (2020), vol. 34, no. 6, pp. 847-850, DOI 10.1080/14786419.2018.1501692<br>abstract, 2. Results and discussion, especially, compound25 | 6 |
| A | abstract, 2. Results and discussion, especially, compound25 | 1-5, 7-8 |
| X | XIAO, S. et al. Chemical constituents of the aerial parts of Schnabelia oligophylla. Chemistry of Natural Compounds. (2017), vol. 53, no. 3, pp. 478-482, DOI 10.1007/s10600-017-2026-z<br>abstract, in particular, compound 6 | 6 |
| A | abstract, in particular, compound 6 | 1-5, 7-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 August 2024** | **27 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No. **PCT/JP2024/020712**

**C. DOCUMENTS CONSIDERED TO BE RELEVANT**

| Category* | Citation of document, with indication, where appropriate, of the relevant passages | Relevant to claim No. |
|---|---|---|
| X | WU, Q. et al. Constituents of Hypericum japonicum. Phytotherapy Research. (1998), 12 (supply 1), S164-S168, DOI 10.1002/(SICI)1099-1573(1998)12:1+<S164::AID-PTR285>3.0.CO:2-G<br>Extraction and isolatioin, RESULT AND DISCUSSION, in particular, compound 1 | 6 |
| A | Extraction and isolatioin, RESULT AND DISCUSSION, in particular, compound 1 | 1-5, 7-8 |
| X | WANG, S. et al. Anti-neuroinflammatory constituents from Artemisia argyi. Journal of Chinese Pharmaceutical Sciences. (2013), vol. 22, no. 4, pp. 377-380, DOI 10.5246/jcps.2013.04.055<br>abstract, fig. 1, compound 4 | 6 |
| A | abstract, fig. 1, compound 4 | 1-5, 7-8 |
| X | LIU, Y. et al. Chemical constituent from the roots of Solanum melongena L. and their potential anti-inflammatory activity. Natural Product Research. (2022), vol. 36, no. 7, pp. 1757-1764, DOI 10.1080/14786419.2020.1815740<br>in particular, compound 8 | 6 |
| A | in particular, compound 8 | 1-5, 7-8 |
| X | ZHOU, Y. et al. A New cyclic octopeptide from Schnabelia oligophylla. Zhiwu Xuebao. (2001), vol. 43, no. 4, pp. 431-434<br>abstract, in particular, compound 2 | 6 |
| A | abstract, in particular, compound 2 | 1-5, 7-8 |
| X | SONG, W. et al. A new lignan from Elaeagnus lanceolata (Elaeagnaceae). Yunnan Zhiwu Yanjiu. (2010), vol. 32, no. 5, pp. 455-462, DOI 10.3724/SP.J.1143.2010.10112<br>in particular, compound 10 | 6 |
| A | in particular, compound 10 | 1-5, 7-8 |
| X | XIANG, W. et al. ent-Kaurene diterpenoids from Isodon adenanthus. Acta Botanica Sinica. (2003), vol. 45, no. 11, pp. 1383-1386<br>abstract, in particular, compound 8 | 6 |
| A | abstract, in particular, compound 8 | 1-5, 7-8 |
| X | LI, J. et al. Improvement Effect of Ficus vasculosa Ethanol Extract on D-galactose-Induced Mice Aging. Natural Product Communications. (2019), vol. 14, no. 12, pp., DOI 10.1177/1934578x19896676<br>abstract, fig. 2, in particular, compound 3 | 6 |
| A | abstract, fig. 2, in particular, compound 3 | 1-5, 7-8 |
| X | YI, P. et al. Three new constituents from the Tujia ethnomedicine Swertia punicea Hemsl. Natural Product Research. (2023), vol. 37, no. 9, pp. 1444-1455, DOI 10.1080/14786419.2021.2012669<br>fig. 2, in particular, compound 40 | 6 |
| A | fig. 2, in particular, compound 40 | 1-5, 7-8 |
| X | XIA, C. et al. Comprehensive Profiling of Macamides and Fatty Acid Derivatives in Maca with Different Postharvest Drying Processes Using UPLC-QTOF-MS. ACS Omega. (2021), vol. 6, no. 38, pp. 24484-24492<br>table 1, fig. 4-5 | 6 |
| A | table 1, fig. 4-5 | 1-5, 7-8 |
| A | WO 2015/174427 A1 (THE KITASATO INSTITUTE) 19 November 2015 (2015-11-19)<br>claims, paragraphs [0001]-[0008], etc. | 1-8 |
| A | US 6787147 B1 (HUNER, Norman) 07 September 2004 (2004-09-07)<br>claims, examples | 1-8 |
| A | JP 04-023880 A (T HASEGAWA CO., LTD.) 28 January 1992 (1992-01-28)<br>claims, examples, etc. | 1-8 |
| A | JP 2014-031361 A (ORIZA YUKA KK) 20 February 2014 (2014-02-20)<br>claims, etc. | 1-8 |

Form PCT/ISA/210 (second sheet) (July 2022)

**INTERNATIONAL SEARCH REPORT**

International application No.
**PCT/JP2024/020712**

**Box No. III Observations where unity of invention is lacking (Continuation of item 3 of first sheet)**

This International Searching Authority found multiple inventions in this international application, as follows:

(Invention 1) Parts of claims 1 and 7, pertaining to an “ultraviolet absorber containing at least one of a compound represented by formula (1) or a compound represented by formula (2) as an active ingredient”

The parts of claims 1 and 7 have the special technical feature of an “ultraviolet absorber containing at least one of a compound represented by formula (1) or a compound represented by formula (2) as an active ingredient," and are thus classified as invention 1.

(Invention 2) Parts of claims 1 and 7, pertaining to an “antioxidant containing at least one of a compound represented by formula (1) or a compound represented by formula (2) as an active ingredient”

It cannot be said that the above-described parts have a special technical feature identical or corresponding to that of the parts classified as invention 1. Also, the above-described parts are not substantially identical to or similarly closely related to any of the parts classified as invention 1.

Therefore, the above-described parts cannot be classified as invention 1, and are thus classified as invention 2.

(Invention 3) Parts of claims 1 and 7, pertaining to an “anti-glycation agent containing at least one of a compound represented by formula (1) or a compound represented by formula (2) as an active ingredient”

It cannot be said that the above-described parts have a special technical feature identical or corresponding to that of the parts classified as invention 1 or 2. Also, the above-described parts are not substantially identical to or similarly closely related to any of the parts classified as invention 1 or 2.

Therefore, the above-described parts cannot be classified as invention 1 or 2, and are thus classified as invention 3.

(Invention 4) Parts of claims 2 and 8, pertaining to a “skin external preparation containing at least one of a compound represented by formula (1) or a compound represented by formula (2) as an active ingredient”

It cannot be said that the above-described parts have a special technical feature identical or corresponding to that of the parts classified as invention 1, 2 or 3. Also, the above-described parts are not substantially identical to or similarly closely related to any of the parts classified as invention 1, 2 or 3.

Therefore, the above-described parts cannot be classified as invention 1, 2 or 3, and are thus classified as invention 4.

(Invention 5) Parts of claims 2 and 8, pertaining to a “cosmetic composition containing at least one of a compound expressed by formula (1) or a compound expressed by formula (2) as an active ingredient”

It cannot be said that the above-described parts have a special technical feature identical or corresponding to the parts classified as any one of inventions 1-4. Also, the above-described parts are not substantially identical to or similarly closely related to any of the parts classified as invention 1, 2, 3 or 4.

Therefore, the above-described parts cannot be classified as any one of inventions 1-4, and are thus classified as invention 5.

(Invention 6) Claims 3-4

It cannot be said that invention 3 has the special technical feature identical or corresponding to those of the parts classified as any one of inventions 1-5. Also, claim 3 is not substantially identical to or similarly closely related to any of parts classified as invention 1, 2, 3, 4, or 5.

Therefore, claim 3 cannot be classified as any one of inventions 1-5, and is thus classified as invention 6.

Furthermore, claim 4 is dependent on claim 3, is inventively related to claim 3, and is thus classified as invention 6.

(Invention 7) Claim 5

There are no other same or corresponding special technical features between claim 5, the parts classified as any one of inventions 1-5, and claims 3-4 classified as invention 6. Also, claim 5 is not substantially identical to or similarly closely related to the parts classified as any of inventions 1-5 and claims 3-4 classified as invention 6.

Therefore, claim 3 cannot be classified as any one of inventions 1-6, and thus the above-described parts are classified as invention 6.

(Invention 8) Claim 6

There are no other same or corresponding special technical features between claim 6, the parts classified as any one of inventions 1-5, claims 3-4 classified as invention 6, and claim 5 classified as invention 7. Also, claim 6 is

Form PCT/ISA/210 (continuation of first sheet) (July 2022)

**INTERNATIONAL SEARCH REPORT**

International application No.
**PCT/JP2024/020712**

**Box No. III Observations where unity of invention is lacking (Continuation of item 3 of first sheet)**

not substantially identical to or similarly closely related to the parts classified as any of inventions 1-5, claims 3-4 classified as invention 6, and claim 5 classified as invention 7.

Therefore, claim 6 cannot be classified as any one of inventions 1-7, and thus the above-described parts are classified as invention 8.

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/020712**

| Patent document cited in search report | Publication date (day/month/year) | Patent family member(s) | Publication date (day/month/year) |
|---|---|---|---|
| WO 2015/174427 A1 | 19 November 2015 | US 2017/0202762 A1<br>claims, paragraphs [0002]-[0008], etc.<br>EP 3144392 A1<br>KR 10-2017-0002587 A<br>CN 106574282 A | |
| US 6787147 B1 | 07 September 2004 | WO 2000/024369 A1<br>CN 1346260 A | |
| JP 04-023880 A | 28 January 1992 | (Family: none) | |
| JP 2014-031361 A | 20 February 2014 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2014031361 A